(19) **Europäisches Patentamt / European Patent Office / Office européen des brevets**

(11) **EP 4 475 746 B1**

(12) **EUROPEAN PATENT SPECIFICATION**

(45) Date of publication and mention
of the grant of the patent:
**01.10.2025  Bulletin 2025/40**

(21) Application number: **22705155.4**

(22) Date of filing: **10.02.2022**

(51) International Patent Classification (IPC):
**A61B 5/021** (2006.01)  **A61B 5/00** (2006.01)

(52) Cooperative Patent Classification (CPC):
**A61B 5/02116; A61B 5/7235; A61B 5/7246**

(86) International application number:
**PCT/IB2022/051194**

(87) International publication number:
**WO 2023/152546 (17.08.2023 Gazette 2023/33)**

(54) **RECONSTRUCTION OF A PATIENT-SPECIFIC CENTRAL ARTERIAL PRESSURE WAVEFORM MORPHOLOGY FROM A DISTAL NON-INVASIVE PRESSURE MEASUREMENT**

REKONSTRUKTION EINER PATIENTENSPEZIFISCHEN MORPHOLOGIE DES ZENTRALEN ARTERIENDRUCKS AUS EINER DISTALEN NICHTINVASIVEN DRUCKMESSUNG

RECONSTRUCTION D'UNE MORPHOLOGIE DE FORME D'ONDE DE PRESSION ARTÉRIELLE CENTRALE SPÉCIFIQUE À UN PATIENT À PARTIR D'UNE MESURE DE PRESSION NON INVASIVE DISTALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**18.12.2024  Bulletin 2024/51**

(73) Proprietor: **HEMOLENS DIAGNOSTICS SPÓLKA Z OGRANICZONA ODPOWIEDZIALNOSCIA**
**54-202 Wroclaw (PL)**

(72) Inventors:
• **MIROTA, Kryspin**
**52-200 Wroclaw (PL)**
• **POPIEL, Izabela**
**53-402 Wroclaw (PL)**

(74) Representative: **JWP Patent & Trademark Attorneys**
**ul. Minska 75**
**03-828 Warszawa (PL)**

(56) References cited:
**WO-A1-2020/048642      US-A1- 2009 287 097**
**US-A1- 2011 137 183      US-A1- 2016 196 384**

**Description**

FIELD OF THE INVENTION

The present invention pertains to an estimation of parameters of a human heart for a subsequent analysis and diagnosis of a human patient. Specifically, the invention pertains to a reconstruction of a central arterial pressure waveform morphology from non-invasive continuous pressure measurements. The reconstructed waveform morphology can be used in diagnosis and treatment of an elevated blood pressure and/or hypertension.

BACKGROUND

In every cardiac cycle, a human heart pumps approximately 100 milliliters of blood into a blood circulatory system. A significant portion of a heart's stroke volume is stored in the elastic arteries, to supply the target regions and organs during the heart relaxation phase. This phenomenon creates a kind of a subtle and dynamic equilibrium, the disturbance of which causes many of serious pathologies including a persistently elevated blood pressure. An elevated blood pressure has some association with hypertension. There are many definitions of hypertension, which can be found in the literature. One of them, provided by the Centers for Disease Control and Prevention, defines hypertension as a systolic blood pressure greater than or equal to 130 mmHg or a diastolic blood pressure greater than or equal to 80 mmHg, or a state in which a patient is currently taking medication to lower high blood pressure (see Ostchega Y (2020). Hypertension Prevalence Among Adults Aged 18 and Over: United States, 2017-2018. NCHS data brief, (364), 1-8.). According to the World Health Organization (WHO) guidelines (2021) hypertension - or an elevated blood pressure - is a serious medical condition that significantly increases the risk of a heart, a brain and/or a kidney disease as well as other diseases. Hypertension can be diagnosed using specific systolic and diastolic blood pressure levels or be indicated by the use of antihypertensive medications (see World Health Organization (2021) Guideline for the pharmacological treatment of hypertension in adults). Another definition can be found in the American College of Cardiology/American Heart Association report. It differentiates two stages of hypertension: stage one is identified when a systolic pressure is within the range of 130-139 mm Hg or a diastolic pressure is within the range of 80-89 mm Hg and stage two is identified when a systolic pressure is equal or above 140 mm Hg or a diastolic pressure is equal or above 90 mm Hg (see Whelton PK (2018) 2017 ACC/ AHA/ AAPA/ ABC/ ACPM/ AGS/ APhA/ ASH/ ASPC/ NMA/ PCNA guideline for the prevention, detection, evaluation, and management of high blood pressure in adults: a report of the American College of Cardiology / American Heart Association Task Force on Clinical Practice Guidelines. Hypertension, 71:e13-e115). A similar explanation is presented by the European Society of Cardiology and the European Society of Hypertension: hypertension is defined as office systolic blood pressure values above 140 mm Hg and/or diastolic values equal or above 90 mm Hg. The same classification is used for young, middle-aged, and older people. However, BP centiles are used for children and teenagers, for whom the data from interventional trials are not available (see Williams B (2018) 2018 ESC/ESH Guidelines for the management of arterial hypertension. European heart journal, 39(33), 3021-3104).

Prevalence of hypertension is linked to genetic (e.g., polygenic influence) and environmental factors (e.g., diet, physical activity, sodium and potassium intake and / or alcohol consumption). Common causes of a secondary hypertension are: a renal parenchymal disease, a renovascular disease, a primary aldosteronism, an obstructive sleep apnea and a drug or an alcohol intake (see Whelton PK (2018) cited above).

Hypertension can result in left ventricular hypertrophy and coronary artery disease (CAD). Left ventricular hypertrophy is caused by pressure overload which results in increased muscle mass and wall thickness without increase in ventricular volume. As the result, a diastolic function becomes impaired, ventricular relaxation is slowed and filling is delayed. Left ventricular hypertrophy can cause sudden death as it is an independent risk factor for cardiovascular disease (see Aronow WS (2017) Hypertension and left ventricular hypertrophy. Annals of Translational Medicine, 5(15), 310). There is no threshold blood pressure value for which complications start to occur. The effects of hypertension are determined by the severity of the condition. An elevated blood pressure is linked to increased morbidity in the whole range of blood pressure. Chronic arterial hypertension accelerates coronary artery disease, leads to myocardial ischemia and myocardial infarction and is an important risk factor for death from CAD. Chronic pressure overload results in heart failure, it starts as a diastolic dysfunction and progresses into an overt systolic failure with a cardiac congestion. A serious implication of hypertension is a stroke that can result from thrombosis, thromboembolism or intracranial hemorrhage. Slowly progressing consequence of hypertension is a renal disease that initially reveals itself in micro-albuminemia to become evident over the years (according to Foëx P (2004) Hypertension: pathophysiology and treatment. Continuing Education in Anaesthesia Critical Care & Pain, 4(3), 71-75).

Hypertension seems to be associated also with common non-cardiovascular diseases including dementia, cancer, osteoporosis and oral health diseases (see Kokubo Y (2015) Higher blood pressure as a risk factor for diseases other than stroke and ischemic heart disease. Hypertension, 66(2), 254-259). It increases the risk of atrial fibrillation (a type of chronic arrhythmia, see Benjamin EJ (1994) Independent risk factors for atrial fibrillation in a population-based cohort.

The Framingham Heart Study. JAMA, 271(11), 840-844), it is responsible for declining glomerular filtration rate and for progression of chronic kidney disease (see Buckalew VM (1996) Prevalence of hypertension in 1,795 subjects with chronic renal disease: the modification of diet in renal disease study baseline cohort. Modification of Diet in Renal Disease Study Group. American journal of kidney diseases: the official journal of the National Kidney Foundation, 28(6), 811-821). Hypertension can lead to changes in blood flow, blood-brain barrier integrity, or to changes in brain in dementia (see Moretti R (2008) Risk factors for vascular dementia: hypotension as a key point. Vascular health and risk management, 4(2), 395-402). The relationship between dementia and high blood pressure in the elderly is controversial. Some studies describe an association between dementia and hypertension (see Gorelick PB (2011) Vascular contributions to cognitive impairment and dementia: A statement for healthcare professionals from the American Heart Association/American Stroke Association. Stroke, 42(9), 2672-2713), when others connect dementia with hypotension (see Novak V (2010) The relationship between blood pressure and cognitive function. Nature reviews. Cardiology, 7(12), 686-698). An increment of blood pressure results in the increased risk of cancer incidents and cancer mortalities (see Stocks T (2012) Blood pressure and risk of cancer incidence and mortality in the Metabolic Syndrome and Cancer Project. Hypertension, 59(4), 802-810). However, additional studies are needed, because of potential interactions between hypertension and other metabolic and carcinogenic factors. It has been observed that a healthy lifestyle reduces the risks both of hypertension and cancer (Kokubo Y (2015) cited above).

Between 2017 and 2018, 45% (value calculated as average of 51.0% of men and 39.7% of women) of U.S. adults had hypertension. Prevalence increased with age - among adults aged 60 over 75% (75.2% men and 73.9% women) of them were diagnosed with hypertension. The disease occurred most frequently in the group of non-Hispanic black men and women (see Ostchega Y (2020) cited above).

According to the WHO, 1.28 billion adults (aged 30-79) worldwide have hypertension, wherein 2/3 of them are living in low- and middle-income countries. Forty-two percent of adults are diagnosed and treated, however 46% of adults are unaware that they have the disease. Only about 1 in 5 have hypertension under control (according to World Health Organization (25 August 2021) Hypertension. https://www.who.int/news-room/fact-sheets/detail/hypertension).

A systolic and a diastolic blood pressure are the most common blood pressure measures used in research studies and clinical practice. They are established independent risk factors of a cardiovascular disease and they can be estimated directly (see Muntner P (2019) Measurement of Blood Pressure in Humans: A Scientific Statement from the American Heart Association. Hypertension, 73(5), e35-e66).

In addition to systolic and diastolic blood pressure values, a pressure waveform contour shape is also important in diagnosis of cardiovascular lesions. A reduced systemic arterial compliance, which is considered to be the best indicator of an impaired pulsatile arterial function, can be detected via analysis of the waveform contour (according to McVeigh GE (1999) Age-related abnormalities in arterial compliance identified by pressure pulse contour analysis: aging and arterial compliance. Hypertension, 33(6), 1392-1398).

A contour analysis of a digital volume pulse provides a simple, non-invasive and reproducible measure of large artery stiffness (see Millasseau SC (2002) Determination of age-related increases in large artery stiffness by digital pulse contour analysis. Clinical Science, 103(4), 371-377). Indices of arterial stiffness that can be measured using waveform analysis are: a pulse wave velocity (which is a pulse velocity along a length of artery), an augmentation index (which is the difference between second and first systolic peaks divided by a pulse pressure), a capacitive compliance (which is a pressure change to volume change ratio during exponential phase of diastolic pressure decay) and an oscillatory compliance (which is an oscillating pressure change to oscillating volume change ratio during diastolic pressure decay exponential phase) (see Mackenzie IS (2002) Assessment of arterial stiffness in clinical practice. QJM: Monthly Journal of the Association of Physicians, 95(2), 67-74).

Similarly, a cardiac output as an important determinant of oxygen delivery that can also be estimated using a continuous pulse wave analysis (see Saugel B (2021) Cardiac output estimation using pulse wave analysis-physiology, algorithms, and technologies: a narrative review. British Journal of Anaesthesia, 126(1), 67-76). Pulse contour analysis is an appropriate method of monitoring a cardiac index after surgery in pediatric patients with a congenital heart disease. There is a strong correlation between cardiac indexes calculated using thermodilution and contour analysis ($r^2 = 0.86$) (see Fakler U (2007) Cardiac index monitoring by pulse contour analysis and thermodilution after pediatric cardiac surgery. The Journal of Thoracic and Cardiovascular Surgery, 133(1), 224-228). An arterial waveform analysis allows calculation of derived parameters like a stroke volume, a cardiac output, a vascular resistance, a stroke volume variation, and a pulse pressure variation (see Esper SA (2014) Arterial waveform analysis. Best Practice & Research. Clinical Anaesthesiology, 28(4), 363-380). Other features that can be extracted from a pressure waveform are MPA and MNA (maximum positive and negative amplitudes, respectively). MPA and MNA when measured on index fingers of both hands using photoplethysmography are important parameters for early-stage cerebral artery stenosis screening (see Kang HG (2018) Identification of Cerebral Artery Stenosis Using Bilateral Photoplethysmography. Journal of Healthcare Engineering, 2018, 3253519).

The risk of coronary and peripheral arterial, cardiopulmonary, cerebrovascular diseases as well as renal diseases can be assessed with a powerful independent risk factor - blood pressure. An accurate measurement is crucial for classification, assessment of blood pressure related risks and for planning treatment (see Pickering TG (2005) Recommendations for blood pressure measurement in humans and experimental animals, part 1: blood pressure measurement in humans: a statement for professionals from the Subcommittee of Professional and Public Education of the American Heart Association Council on High Blood Pressure Research. Hypertension, 45:142-161). Evidence of a relationship between a cardiovascular disease and a blood pressure are provided in many epidemiological studies. According to estimates, individuals with prehypertension account for ~15% of blood pressure related fatalities caused by coronary heart disease (see Miura K (2001) Relationship of blood pressure to 25-year mortality due to coronary heart disease, cardiovascular diseases, and all causes in young adult men: the Chicago Heart Association Detection Project in Industry. Archives of Internal Medicine, 161(12), 1501-1508). Variability of blood pressure is the principle rather than the exception.

For nearly 100 years the main method of measuring blood pressure was the auscultatory method. Despite its limited accuracy, the Korotkoff method has been used without any significant improvement. However, the method is being replaced by new methods that are more suitable for automatic measurements.

The gold standard for clinical measurements has traditionally been the mercury sphygmomanometer. Its implementation has not altered much over the last 50 years, with the exception that newer ones are better protected against the spillage of mercury if dropped. Aneroid sphygmomanometers use a mechanical system of metal bellows and levers. Disadvantage of this system is losing stability and a need for regular calibration. Aneroid sphygmomanometers accuracy is lower than mercury sphygmomanometer and it varies widely between manufacturers. Hybrid Sphygmomanometers are devices combining properties of both auscultatory and electronic devices. A mercury column is replaced by an electronic pressure gauge, like these used in oscillometers. Hybrid devices have a potential to replace mercury based devices when electronic devices become more accurate (see Pickering TG (2005) cited above).

Next method of a blood pressure measurement is an oscillometric technique which was first presented in 1876 by Marey (see Marey EJ (1876) Physiologie expérimentale: Travaux du Laboratoire de M. Marey). Further observations on the method showed that the point of maximal oscillation in a cuff measurement corresponds to the mean intra-arterial pressure when recorded during gradual deflation (see Mauck G (1980) The meaning of the point of maximum oscillations in cuff pressure in the indirect measurement of blood pressure--Part II. Journal of Biomechanical Engineering, 102(1), 28-33).

The oscillations begin above systolic pressure and proceed below diastolic. As the result, systolic and diastolic pressures are evaluated indirectly using algorithm based on empirical data. Method's advantages are that cuff placement is not essential - there is no need to place a transducer over the brachial artery, it is less sensitive to external noise and a cuff is removable (see Pickering TG (2005) cited above).

Limitation of the oscillometric method results from the fact that oscillations depend not only on blood pressure but also on several other factors, among which the most important factor is artery stiffness. Among older people with stiff arteries the value of mean arterial pressure can be underestimated. Also, algorithms detecting diastolic and systolic pressures are not published by manufacturers and that results in significant measurement differences when using devices from different manufacturers (see Amoore JN (2000) Can simulators evaluate systematic differences between oscillometric non-invasive blood-pressure monitors? Blood pressure monitoring, 5(2), 81-89). Oscillometric devices show good enough agreement with intraarterial and Korotkoff method measurement. They are also cheaper in comparison the devices used in intraarterial and Korotkoff method and that makes them suitable for application in both ambulatory and home monitoring (see Pickering TG (2005) cited above).

Another method is the finger cuff method using "unloaded arterial wall" principle. Arterial pulsation is detected in a finger by a photoplethysmograph under a pressure cuff. The cuff is inflated to the same pressure as is in the artery and further until it is about to collapse and the transmural pressure is near zero. A photoplethysmograph's output is used in a servomechanism system, that controls cuff pressure (see Muntner P (2019) cited above). This kind of solution was presented by Peňáz. In addition to the finger arteries, measurements are also possible on other arteries which can be easily transilluminated (accessible from the surface, lying in a soft tissue against a background e.g., bone). Typical examples are forearm or temporal region (Peňáz J (1988) Automatic noninvasive blood pressure monitor. U.S. Patent no. 4,869,261). In general, cuff oscillometric measurements provide information only about systolic and diastolic pressure values.

The last group are methods using tonometry. The principle of these methods is based on compressing or splinting an artery against a bone, so resulting pulsation is proportional to an intra-arterial pressure. A method can be used to measure pressure signal at a wrist where the radial artery lies over the radius bone. The measured signal is position-sensitive, so the transducer needs to be located directly over the artery's center. The method is not appropriate for use in an ordinary clinical practice, because of a need for calibration for each patient. In the applanation tonometry, a pressure waveform is recorded over the radial artery with a single transducer held manually. The brachial artery is used to monitor systolic and diastolic pressures (see Pickering TG (2005) cited above).

A blood pressure should be measured in each arm during the initial appointment to select the arm with a higher blood pressure value. Follow-up appointments should measure blood pressure using the arm selected during the initial appointment (see Williams B (2018) 2018 ESC/ESH Guidelines for the management of arterial hypertension. European Heart Journal, 39(33), 3021-3104). However, there is no clear pattern in differences between the two arms, but it was observed that differences of about 10 mm Hg occurred in 20% of subjects (according to Lane D (2002) Inter-arm differences in blood pressure: when are they clinically significant? Journal of Hypertension, 20(6), 1089-1095). It is recommended to use a cuff with a bladder length that is 80% and width that is at least 40% of arm circumference. Initially, a cuff should be inflated to at least 30 mm Hg above the point of radial pulse disappearance. Deflation rate should be 2 to 3 mm Hg per second (see Pickering TG (2005) cited above). The measurement is commonly made while sitting or in s supine position and that alternative gives different results. While sitting, diastolic pressure measured is about 5 mm Hg higher than when measured in a supine (see Netea RT (2003) Influence of body and arm position on blood pressure readings: an overview. Journal of Hypertension, 21(2), 237-241). Systolic pressure is 8 mm Hg higher in the supine position than in the upright position when a cuff is at the level of the right atrium (see Terént A (1994) Epidemiological perspective of body position and arm level in blood pressure measurement. Blood pressure, 3(3), 156-163). Diastolic pressure can be 6 mm Hg greater when patient's back is not supported (following Cushman, 1990). Leg crossing can result in 2 to 8 mm Hg systolic pressure raise (Peters GL (1999) The effect of crossing legs on blood pressure: a randomized single-blind cross-over study. Blood pressure monitoring, 4(2), 97-101). All these considerations show an ambiguity of results obtained via tonometry methods. At best, accuracy of these methods depends on experience of a person carrying out measurements, even if a waveform contour can be obtained using the applanation tonometry.

Taking into account all previously mentioned methods one can see their limitations. In this case, a noninvasive blood pressure (NIBP) monitoring seems to be the optimal waveform measurement for the clinical purposes. NIBP devices can use a finger cuff with a photoplethysmograph or implement a type of applanation tonometry in which the sensor is not held during measurement but placed in e.g., a bracelet, to avoid user bias (see Lakhal K (2018). Noninvasive BP Monitoring in the Critically Ill: Time to Abandon the Arterial Catheter? Chest, 153(4), 1023-1039).

There are three main strategies for deriving central artery pressure from radial measurements, namely: a spectral (frequency) domain representation of signals and resynthesis, a variation of autoregressive moving average model, and finally reduced-order or lumped-parameter models.

Spectral representation (sometimes also called frequency approach of determining central from radial pressure) was directly derived from the theory of signal analysis and processing. The method was formulated by Mustafa Karamanoglu co-workers from the Australian University of New South Wales in the early 1990s (see Karamanoglu (1993) M An analysis of the relationship between central aortic and peripheral upper limb pressure waves in man. European Heart Journal. Feb;14(2):160-7). Subjects of the experiment were 14 patients. For each of them brachial and ascending aorta pressure waves were recorded by micromanometer, and a radial artery pressure was recorded by the applanation tonometry. Measurements of pressure waves were made at steady state conditions before and after administration of a nitroglycerine tablet. Transfer functions were obtained using Fourier analysis and each transfer function was defined by the following equation:

$$H_{(A-B)} = \frac{P_B(\omega)}{P_A(\omega)} \, , \hspace{4cm} (1)$$

where $P_A(\omega)$ and $P_B(\omega)$ are frequency representations of ascending aorta and brachial or artery pressures, respectively. Using a modulus and a phase of a pressure signal representation, a transfer function was transformed into:

$$H_{(A-B)} = \frac{M_B(\omega)e^{j\varphi_B(\omega)}}{M_A(\omega)e^{j\varphi_A(\omega)}} = M_{A-B}(\omega)e^{j\varphi_{A-B}(\omega)} \, , \hspace{2cm} (2)$$

where $M_{A-B}(\omega) = P_B(\omega)/P_A(\omega)$ is the modulus and $e^{j\varphi_{A-B}(\omega)} = e^{j\varphi_B(\omega)} - e^{j\varphi_A(\omega)}$ is the phase of the transfer function. A generalized transfer function was calculated by pooling individual transfer functions and averaging both modulus and phase values inside bins of 1 Hz and its multiplies. A reconstruction of an aortic pressure from a brachial or a radial pressure comprised of transforming a peripheral pressure into the frequency domain using a discrete Fourier transform and dividing its harmonic contents by harmonic contents of the transfer function. The results were retransformed to the time domain by an inverse discrete Fourier transform:

$$P_{A'}(\omega) = \frac{P_B(\omega)}{GTF(\omega)} \, , \hspace{4cm} (3a)$$

$$P_{A'} = \mathcal{F}^{-1}\big(P_{A'}(\omega)\big) . \hspace{4cm} (3b)$$

Contour and amplitude of pressure waves in the ascending aorta compared to pressure waves in the brachial and radial arteries were significantly different. After administration of nitroglycerine differences were even greater. Despite contour differences, harmonic components of the transfer function were similar. For the pooled data, the modulus and the phase did not differ substantial between those from control conditions and those obtained after administration of nitroglycerine. To obtain a transfer function that works under different conditions, transfer functions for nitroglycerine were averaged with transfer functions for control conditions. The obtained averaged transfer function was similar to those calculated for control conditions and following administration of nitroglycerine. According to the authors' own results, the credibility of the method is relatively high. The accuracy of central pressure reconstruction was assessed using systolic pressure values. A comparison between recorded aortic and peripheral systolic pressures showed significant differences ($r^2 = 0.89$). A comparison of synthesized and directly recorded aortic systolic pressures under the different conditions of measurement showed minor differences ($r^2 = 0.95$).

This first device to use this method was SphygmoCor® which was considered to be a standard for central blood pressure estimation. Although, validation studies accepted estimation accuracy for this device only for estimations of a central systolic blood pressure and a central pulse pressure. The method allowed to obtain a full waveform but it was not precise enough to conduct a complete waveform contour analysis (according to Hope SA (2007) 'Generalizability' of a radial-aortic transfer function for the derivation of central aortic waveform parameters. Journal of Hypertension, 25(9), 1812-1820).

The models comprised in the second group (called Autoregressive moving average model-type, ARMA-type) were first proposed and popularized through the work of Chen-Huan Chen and co-authors (team from Baltimore's Johns Hopkins University Medical Institution) at the end of the nineties (see Chen CH (1997) Estimation of central aortic pressure waveform by mathematical transformation of radial tonometry pressure. Validation of generalized transfer function. Circulation. Apr 1,95(7):1827-36). The basic concept of these models is based on calculating individual transfer functions (TFs) between pairs of central and radial aortic pressures using the ARMAX (AutoRegressive Moving Average with eXogenous input) model and generalizing individual TFs to obtain a generalized transfer function (GTF). The main allegation against spectral method was that interpatient and intrapatient variability of the TFs was not systematically assessed. Chen's goals were to determine the magnitude of TFs variations between central and radial pressure waves both under control conditions and physiological maneuvers that heavily altered blood pressure.

For each of 20 patients, aortic pressures were recorded with a micromanometer, and radial pressures were recorded with an automated tonometry device. Data recordings were made at steady state in each subject and then during at least one of several hemodynamic transient maneuvers. TFs between aortic and radial pressures were calculated for each subject using the linear ARMAX model. Direct TFs corresponding to a physiological system were derived with an aortic pressure input and a radial tonometer signal output:

$$T(t) = -a_1 T(t-1) - a_2 T(t-2) - \cdots - a_{na} T(t-na) + b_1 P(t-1) + \cdots + b_{nb} P(t-nb)\,, \qquad (4)$$

where T(t) and T(t-I) [I=1,2...na] were present and previous radial discrete measurements and P(t-I) were previous aortic discrete measurements. Parameters of the model were a, b values and the order was represented by na, nb. In this study the order was set arbitrary to [10,10].

To enable aortic pressure reconstruction from a radial signal, a direct TF was inversed:

$$P(t-1) = -\frac{b_2}{b_1 P(t-2)} - \cdots \frac{b_{nb}}{b_1 P(t-nb)} + \frac{1}{b_1 T(t)} + \frac{a_1}{b_1 T(t-1)} + \cdots + \frac{a_{na}}{b_1 T(t-na)}\,. \qquad (5)$$

ARMAX parametric models were compared with a nonparametric method - a spectral TF estimation obtained with a Fourier transform. This parametric model produced estimations that had smaller variance when compared to those obtained using a nonparametric model for the same dataset. A variance for both methods was similar for larger datasets. Finally, only a generalized steady state inverse transfer function was used for a waveform resynthesis analysis, because the steady-state and the transient generalized transfer function were similar.

A pulse amplitude and a contour of the estimated waveforms at steady state were similar to the measured central waveforms when the estimated waveforms were obtained using individual or generalized inverse transfer functions. An individual function resulted in a slightly greater accuracy in a waveform estimation (said accuracy was compared using measurements of a minimal area for the regressions of the estimated and the measured waveforms plots). The calculated central arterial pressures differed from the measured values by $\leq 0.2 \pm 3.8$ mm Hg when the calculations were made using a generalized transfer function. The variance for an individual transfer function (ITF) was 0.9 mmHg.

A comparison of augmentation indexes (AI) revealed an important difference as AI values from the reconstructed waves were lower than the ones calculated based on the measured waveforms. A GTF resulted in $30 \pm 45\%$ lower AI values and the use of ITF reduced variance in this underestimation.

A resynthesis of aortic waveforms under transient loading change depended on a transfer function constancy, both for a GTF and an ITF. On average the TF was constant, although several patients displayed marked changes in a TF during the transient, so pressure could not be accurately reconstructed. This degree of intrapatient variability of a TF (coefficient of variation for peak amplitude >20%) occurred in 4 of 14 subjects. The authors' conclusion was that a GTF produced nearly as reliable results as an ITF. This implies that a vascular branching in the upper extremity that results in a pressure amplification is a much stronger factor affecting a TF when compared to factors like age, sex or body morphology.

There is also a similar method - the n-point moving average (NPMA) that works as a digital low-pass filter used for smoothing waveforms and for eliminating a high-frequency noise. However, the optimal moving average denominator for the method is determined empirically using validation data from a selected population. As the result, the method's accuracy cannot be better than obtained for the spectral method (following Miyashita H (2012) Clinical Assessment of Central Blood Pressure. Current hypertension reviews, 8(2), 80-90).

The third group of methods of determining an aortic pressure wave was based a tube load models proposed either by Mukkamala (see R Mukkamala (2019) Methods and apparatus for determining a central aortic pressure waveform from a peripheral artery pressure waveform. U.S. Patent No. 10,251,566 B2) or by Gao (see Gao M (2016) A simple adaptive transfer function for deriving the central blood pressure waveform from a radial blood pressure waveform. Scientific Reports, 6(1), 1-9). The following description is about the methods using Mukkamala approach, which is a bit more elaborated. A mathematical transformation between aortic (AP) and peripheral (PAP) pressure waveforms was built on a distributed model representing an arterial tree and on an assumption of a negligible central aortic flow during the diastole due to aortic valve closure. The first step was to use the distributed model to define a transfer function between a PAP and an AP, and between a PAP and a central arterial flow. Parameters of the model were estimated by finding another transfer function which when applied to the measured PAP would minimize the magnitude of a central arterial flow waveform under the diastole conditions. Those parameters were substituted in the former transfer function to finally transform a PAP to an AP. A transfer function was updating its parameters every time when a new waveform segment became available.

A distributed model representing the arterial tree comprised of parallel segments made of uniform tubes (paths between the aorta and the peripheral artery) arranged in series with a lumped parameter terminal load (arterial bed distal to peripheral artery). A pressure to pressure transfer function is given with the following equation:

$$\frac{\left(\frac{B_i + A_i}{2} + j\omega\right) e^{j\omega T_{di}} + \left(\frac{B_i - A_i}{2}\right) e^{-j\omega T_{di}}}{B_i + j\omega} \, . \tag{6}$$

A pressure to flow transfer function is given below:

$$\frac{\left(\frac{B_i + A_i}{2} + j\omega\right) e^{j\omega T_{di}} + \left(\frac{B_i - A_i}{2}\right) e^{-j\omega T_{di}}}{Z_{ci}(B_i + j\omega)} \, . \tag{7}$$

The unknown parameters $T_{di}$, $A_i$ and $B_i$ were calculated for every segment of a PAP waveform using the fact that the central aortic flow during the diastole is insignificant. Parameters of pressure to flow transfer function were determined using measured PAP and taking "0" as value of the central arterial flow during a diastolic phase. A parameter estimation was simplified using non-invasively measured values of $T_{di}$. Form each PAP waveform segment and initial (measured) $T_{di}$ values, three parameters of pressure-to-flow functions were approximated.

Prior art search revealed papers presenting a clinical evaluation of described non-invasive methods of hemodynamic monitoring. One of the papers compared a non-invasive measurement of arterial pressure made with ClearSight™ (a vascular unloading technique) with an invasive measurement made following induction of anesthesia during a cardiac surgery, so at the time when the measurement of a mean arterial pressure is needed. The conclusion was that even considering the limitations like the blue-finger syndrome, the non-invasive measurement was valuable alternative. The use of the non-invasive measurement was a viable alternative to an invasive measurement especially for anxious patients or when there is an expectation for difficult conditions in the form of a radial artery puncture (see Frank P (2021) Noninvasive continuous arterial pressure monitoring during anesthesia induction in patients undergoing cardiac surgery. Annals of Cardiac Anaesthesia, 24:281-7). The opposite position was presented in a review article by Kim (see Kim SH (2014) Accuracy and precision of continuous noninvasive arterial pressure monitoring compared with invasive arterial pressure: a systematic review and meta-analysis. Anesthesiology, 120(5), 1080-1097). The researchers conducted a comprehensive review and a meta-analysis that comprise a comparison of a continuous non-invasive with an invasive arterial pressure monitoring. Continuous non-invasive monitoring results were accepted and recommended by the Association for the Advancement of Medical Instrumentation if pooled estimates of bias and a standard deviation were not higher than 5 and 8 mmHg, respectively. Findings showed that inaccuracy and imprecision of continuous non-invasive arterial pressure monitoring devices were higher than accepted. It should be noted that the purpose of the study was to assess the relative accuracy of the non-invasive monitoring and not the potential clinical utility of devices that used said monitoring. After all, a clinical decision-making process, the final outcome or safety requires more an accurate and precise measuring device. Another metanalysis (see Saugel B (2020) Continuous noninvasive pulse wave analysis using finger cuff technologies for arterial blood pressure and cardiac output monitoring in perioperative and intensive care medicine: a systematic review and meta-analysis. British Journal of Anaesthesia, 125(1), 25-37.) confirmed the results of the previous one. This analysis comprised the results of several studies and said results accepted interchangeability of an arterial pressure, a cardiac output and a cardiac index when measured using non-invasive finger cuff devices and invasive methods as reference. However, pooled results presented in this meta-analysis showed that non-invasive and invasive methods are not interchangeable. This was due to a significant heterogeneity in studies which was embodied by a substantial variability of arterial pressure, cardiac output and cardiac index values. In general, heterogeneity of results is caused by several factors related to a patient (e.g., different populations), a clinical setting (e.g., the use of vasopressors and inotropes) and a device (e.g., different software versions used for monitoring). This review analyzed only the agreement between study and reference methods results and not the trending ability which can be understood as relative changes of an arterial pressure or a cardiac output and an index over time. There are also separate papers on the clinical evaluation of the CNAP™ device (Continuous Noninvasive Arterial Pressure; CNSystems Medizintechnik AG). According to one of them (see Ilies C (2012) Investigation of the agreement of a continuous non-invasive arterial pressure device in comparison with invasive radial artery measurement. British Journal of Anaesthesia, 108(2), 202-210.) under normotensive conditions CNAP™ was interchangeable with an invasive pressure monitoring for mean arterial pressure values. The results were different following induction of anesthesia - an arterial pressure was low and interchangeability was not achieved. The conclusion was that CNAP™ could not be statistically equivalent to invasive methods under anesthesia conditions but could be used as an additional pressure monitoring device. Similar conclusions can be found in an article by R. Hahn (see Hahn R (2012) Clinical validation of a continuous non-invasive haemodynamic monitor (CNAP™ 500) during general anaesthesia. British Journal of Anaesthesia, 108(4), 581-585.) - the CNAP™ monitoring device revealed a promising agreement with an invasive device for an arterial pressure, but the non-invasive device did not meet the predefined requirements.

The cited prior art documents confirm the inexact agreement between invasive and non-invasive measurements. Because of the many advantages of non-invasive measurements (e.g., safety and ease of use), it is necessary to develop mathematical methods to convert a pressure obtained non-invasively to the one obtained invasively.

As the above prior art reviews show, there are many different proposals for extrapolation of the result of distal-to-proximal (central) pressure measurements. In the most traditional approach, these are black-box methods - well established and most widespread in practical applications. They promise to solve the problem via a single, simple, and universal formula regardless of gender, age or other health state specific factors. Their opposite is complex distributed models, with dozens of unknown empirical parameters (which are, more or less, related to physiology).

The current models of heart-vessel interaction include high-dimensional models (e.g., 2D and 3D) as well as low-dimensional models (e.g., 1D, 0D, and tube-load) (see Zhou S, et al. A review on low-dimensional physics-based models of systemic arteries: application to estimation of central aortic pressure. Biomedical Engineering Online. 2019;18(1):41,1-25). The high-dimensional models are rather predestined to the description of a local phenomena in a specific circulatory region. These models are most often derived from mass and balance equations in the Euler and Navier-Stokes form, sometimes complemented with constitutive relations of a vascular tissue (see Morris PD, et al Computational fluid dynamics modelling in cardiovascular medicine, Heart 2016;102:18-28). Due to complexity the high-dimensional models and due to the demand for computing power, the high-dimensional models can only be used to strictly limited circulatory regions. For a broader application, the high-dimensional models need to be simplified. This is done in steps. In the first step, the spatial dimensions are reduced, which leads to a one-dimensional (1D) model (see Raines JK, Jaffrin MY, Shapiro AH. A computer simulation of arterial dynamics in the human leg. Journal of Biomechanics. 1974;7(1):77-91, Formaggia L, Lamponi D, Tuveri M, Veneziani A. Numerical modeling of 1D arterial networks coupled with a lumped parameters description of the heart. Computer Methods in Biomechanics and Biomedical Engineering. 2006; 9(5):273-288) and discontinuous in space tube-load models (see Swamy G, Mukkamala R, Olivier N. Estimation of the aortic pressure waveform from a peripheral artery pressure waveform via an adaptive transfer function. Annual International Conference of the IEEE Engineering in Medicine and Biology Society. 2008:1385-1388).

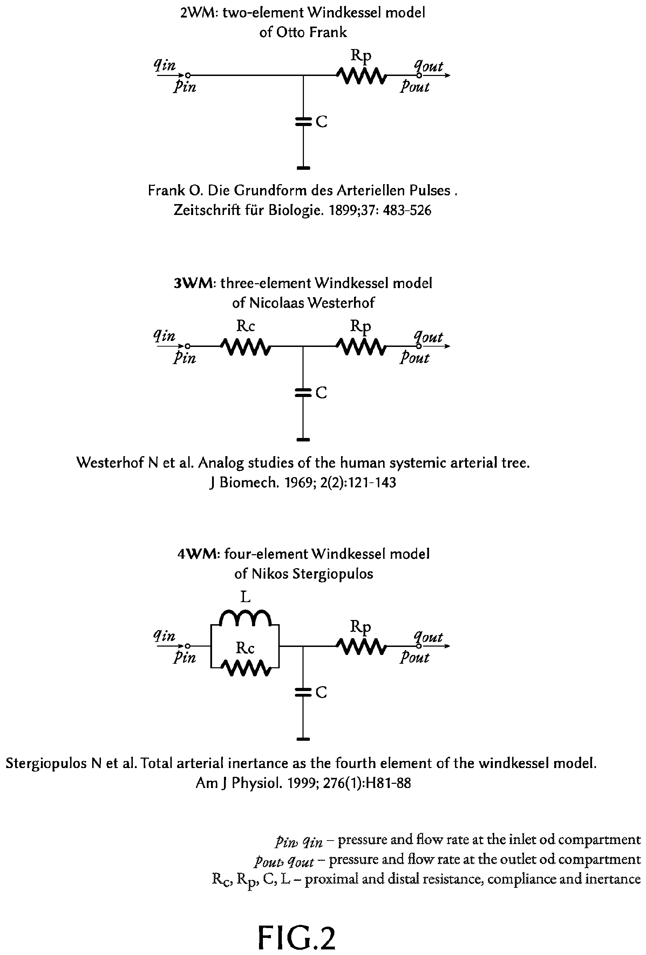

The models with lumped parameters provide a very effective method of description of a key phenomenon observed in the cardiovascular dynamics despite their simplicity (following Vlachopoulos Ch, O'Rourke M, Nichols WW. McDonald's Blood Flow in Arteries: Theoretical, Experimental and Clinical Principles 6th Edition, CRC Press, 2011). Examples of said models are given in FIG. 2. The diagrams in FIG. 2 illustrate each of the three basic configurations of the Windkessel model. The first and the earliest one - published at the end of the nineteenth century by German physiologist Otto Frank - contains only two components (FIG. 2 (2WM)) in the form of compliance ($C = dV/dP$, ratio of volume change to intravascular pressure change) and resistance ($R_p$ as mean pressure to mean flow), modeling distensibility large arteries and resistivity of small peripheral vessels (Frank O. Die Grundform des Arteriellen Pulses. Zeitschrift für Biologie. 1899;37: 483-526). Despite its simplicity, this model is suitable for describing a pressure decay in the aorta and can be used to estimate a cardiac output, or a blood pressure. In order to extend the scope of 2WM applicability to the high frequency range, Nicolaas Westerhof proposed in 1969 addition of a third component - the input inertance (see Westerhof N et al. Analog studies of the human systemic arterial tree. Journal of Biomechanics. 1969; 2(2):121-143). The three-element Windkessel (FIG. 2 (3WM)) was able to reproduce realistic pressure and flow waveforms, as well was able to fit experimental data from *in vivo* measurements. Probably, the 3WM model of Westerhof has become the most widely used and accepted one for the description of the systemic circulation.

The 3WM model neglects inertial effects which was demonstrated by Nikos Stergiopulos in the late nineties of 20th century and that is its main drawback (see Stergiopulos N, Westerhof BE, Westerhof N. Total arterial inertance as the fourth element of the Windkessel model. Am J Physiol. 1999; 276(1):H81-88. It was shown that rapid changes in a pressure in the central artery, which were initially manifested by a rapid acceleration of blood masses in the systolic phase and then their deceleration in the diastole, must lead to inertial effects. In order to take these effects into the account - as Nikos Stergiopulos argued - the model needs to be completed with an additional inertial term $(\Delta p \sim dq/dt)$ (FIG. 2 (4WM)).

This in-depth prior art review highlights the need for an accurate non-invasive method that uses a model which takes into consideration all relevant effects, which is patient-specific and which practical implementation allows the method to be used during e.g., clinical intensive care monitoring. The present invention addresses this need.

US 2016/196384 relates to a cardiovascular system that is composed of cardiac electro-mechanics model coupled to a whole-body circulation model. The system requires acquisition of cardiovascular images, signals and data, including at least one medical image of a patient, acquisition of EKG, and systolic and diastolic cuff pressures. Heart geometry is built using images. The model requires measurements of pressure data and the pressure data includes pressures measured with a pressure cuff. Parameters for a lumped-parameter model are personalized using measured data or estimated directly with measured data.

US 2009/287097 measures a pressure waveform with a supra-systolic cuff and estimates from that measurement a central pressure waveform. This document is relating to a waveform-to-waveform transformation model and uses a modified acoustic wave equation. This allows a suprasystolic waveform to be used in reconstruction of an aortic pressure waveform with using two parameters.

US 2011/137183 objective is to reconstruct a blood pressure waveform from a peripheral blood pressure waveform. This is done with the use of a transfer function that transforms one waveform into another. Transfer (reconstruction) requires the use of a pre-selected parameter which includes measured values, for example, of a heart rate. The peripheral blood pressure waveform is measured non-invasively using, for example, a finger cuff.

WO 2020/048642 relates to calculating hemodynamic parameters associated with coronary arteries. This is achieved with anatomical data that is used to generate an anatomical model and with a continuously recorded blood pressure waveform which is used to determine boundary conditions for a computational fluid dynamics (CFD) simulation of blood flow in the anatomical model.

BRIEF DESCRIPTION OF THE INVENTION

The forthcoming description is to better understand the principle and benefits of the invention claimed in the appended claims. Therefore, it is not meant to be limiting in any sense.

The purpose of the invention is to provide the information about the parameters of a waveform of a central arterial pressure as a function of time using one or more non-invasive measurements. The provided information is corresponding to the values obtained via invasive measurements. Additionally, the invention aims to provide the pressure waveform based on non-invasive measurements wherein said pressure waveform corresponds to the waveform obtained via invasive measurements. Additionally, the invention aims to provide a contour analysis of a digital volume pulse and a cardiac output based on one or more non-invasive measurements wherein said analysis, pulse and output correspond to the ones obtained using invasive measurements.

Thereby, the invention allows to diagnose the condition of a human heart based solely on one or more non-invasive measurements and thus without the risks that are associated with organization and conducting invasive measurements. This allows for diagnosis and treatment of an elevated blood pressure and/or hypertension.

The invention is based on a specific approach of transformation of a measured distal pressure waveform to a proximal (central) pressure waveform done using an inventive model of the Windkessel type.

The one of the key points of the present invention is causality: a distal pressure such as a radial pressure is an effect of a central aortic variability and not the other way around. Despite the fact that the distal pressure is measured, the starting point for building a relationship must be a human heart and major blood vessels connected to the heart. Another key point is the use of models based on patient-specific data and not following universal formulas that do not take into consideration factors like gender, age or other health state specific factors. In particular the invention is not following models based on unknown empirical parameters which are in principle used only for obtaining a better fit to experimental data.

In one aspect, the invention pertains to a method of reconstruction of a central arterial pressure waveform morphology from a distal non-invasive continuous pressure measurement. The method can be realized as a computer-implemented invention.

The method according to the invention comprises gathering of patient-specific demographic and health data which affect pressure pulse propagation in the body. Patient-specific data should be understood as data obtained from a specific human patient. Patient-specific demographic and health data may include patient's gender, age, body height, general fitness assessment and/or current medication. The current medication includes, but is not limited to, a beta-adrenergic blocking agent, an angiotensin-converting-enzyme inhibitor and/or an antiarrhythmic agent. In general, all medications that may affect a human heart can be consider.

The method according to an embodiment that is not claimed herein also comprises a non-invasive measurement or measurements of a patient's systolic pressure, a patient's diastolic pressure, and a patient's heart rate. The term "non-invasive measurement" means a measurement that does not involve any type of surgery and/or significant health risks. In some embodiments, said measurement does not include insertion of any probe into a patient's body. In a certain embodiment that is claimed herein, the method does not require said measurement or measurements. Instead, the method as claimed herein comprises assumption of a patient's systolic pressure, a patient's diastolic pressure, and a patient's heart rate based on a continuously registered patient's distal pressure waveform.

The method according to the invention also comprises continuously registering of a patient's distal pressure waveform e.g., from the distal artery, wherein said registration comprises an entire cycle of patient's heart and wherein registration is done non-invasively. In extreme cases of an abnormally slow breathing rate (bradypnea), a half of a respiratory cycle length can be used. This registration may involve measuring of an arterial blood pressure by a sensor positioned above the radial artery using methods selected from photoplethysmography and/or applanation tonometry. Additionally, this registration may be made over a sequence of consecutive cycles of a heart that are within a single respiratory cycle. This registration may also be done over any number of heart or respiratory cycles. The term "non-invasive registration" means a registration that does not involve any type of surgery and/or significant health risks. In some embodiments, said registration does not include insertion of any probe into a patient's body.

The method according to the invention also comprises performing parametric identification of a coupled system, wherein the system comprises a lumped-parameter model of a central compartment of blood circulatory system and a lumped-parameter model that is responsible for a distal-to-proximal transfer. The two-model structure of the coupled system ensures that transformation of a measured distal pressure to a proximal (central) pressure is done accurately and the obtained results can be used in subsequent analysis and diagnosis of a human heart with precision approaching the results obtained using invasive measurements.

Further characteristics and advantages will be more apparent from the detailed description of non-limiting embodiments and from the appended figures. It should be understood that all embodiments and all features thereof, presented in the description and in the claims, unless stated otherwise, can be combined together in any order and number to produce new embodiments that form part of the disclosure.

BRIEF DESCRIPTION OF FIGURES

The invention will now be described in more detail with reference to the appended figures in which:

FIG. 1 presents a general view of a method according to an embodiment that is not claimed herein,

FIG. 2 presents a schematic block-diagram representation of mono-compartment lumped-parameter circulation model basic variants: (2WM) two-element Otto Frank, (3WM) three-element Nicolaas Westerhof, and (4WM) four-element Nikos Stergiopulos,

FIG. 3 presents a functional building block mono-compartment (CRL) and a generalized form of a multi-compartment (n-CRL) Windkessel model according to preferred embodiments of the invention,

FIG. 4 presents lumped-parameter models of a central compartment of blood circulatory system and functional building blocks,

FIG. 5 presents two exemplary complete recordings of continuous blood pressure waveform measurements in patients which were obtained in clinical trials using an invasive method and obtained using a non-invasive method according to the invention,

FIG. 6 presents a reconstruction of a central arterial pressure waveform vs reference signal from an intra-aortic catheter in the resting and the hyperemic state - a window of five cycles,

FIG. 7 presents a process of convergence of model parameters with the use of local and global minimizing algorithms according to embodiments of the invention, and

FIG. 8 presents an assessment of accuracy of systolic and diastolic pressures reconstructions obtained using an embodiment of a method according to the invention.

DETAILED DESCRIPTION

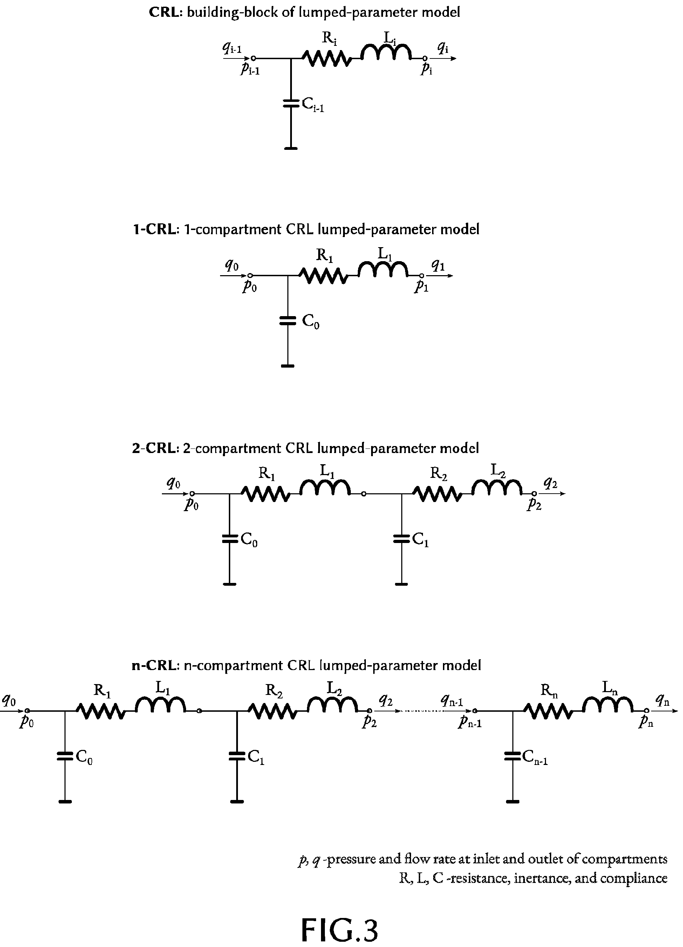

In a preferred embodiment of the invention, reconstruction of a central arterial pressure waveform from a distal non-invasive measurement is performed on the basis of the lumped-parameter multi-compartment model blocks presented in FIG. 3. As shown in the figure, the multi-compartment model is built using CRL functional blocks (CRL - compliance ($C_i$), resistance ($R_i$), and inertance ($L_i$)). The input and output parameters are connected by a pair of equations for pressures *(p)* and flow rates *(q)*:

$$q_{i-1} = C_{i-1}\frac{dp_{i-1}}{dt} + q_i \xrightarrow{yields} \Delta q_i = q_{i-1} - q_i = C_{i-1}\frac{dp_{i-1}}{dt} \,, \tag{8}$$

$$p_{i-1} = R_i q_i + L_i\frac{dq_i}{dt} + p_i \xrightarrow{yields} \Delta p_i = p_{i-1} - p_i = R_i q_i + L_i\frac{dq_i}{dt} \,, \tag{9}$$

therefore, for a single CRL functional block we have the following equation:

$$p_{i-1} = R_i\left(q_{i-1} - C_{i-1}\frac{dp_{i-1}}{dt}\right) + L_i\frac{d}{dt}\left(q_{i-1} - C_{i-1}\frac{dp_{i-1}}{dt}\right) + p_i \,. \tag{10}$$

Thus, for a n-CRL blockchain we obtain the following equations:

$$q_0 = C_0\frac{dp_0}{dt} + q_1, q_1 = C_1\frac{dp_1}{dt} + q_2, \dots, q_{n-1} = C_{n-1}\frac{dp_{n-1}}{dt} + q_n \,, \tag{11}$$

$$p_0 = R_1 q_1 + L_1\frac{dq_1}{dt} + p_1, p_1 = R_2 q_2 + L_2\frac{dq_2}{dt} + p_2, \dots, p_{n-1} = R_n q_n + L_n\frac{dq_n}{dt} + p_n \,. \tag{12}$$

All of the above leads to a more general conclusion that proximal and distal pressures are always bound which can be expressed by:

$$\underbrace{p_0}_{proximal} = \underbrace{\sum_{i=1}^{n-1}\Delta p_i}_{\Delta p} + \underbrace{p_n}_{distal} \,, \tag{13}$$

where $\Delta p$ requires calculation in order to obtain a distal-to-proximal transformation.

The problem of determining of a proximal (central aortic) pressure using a distal (usually radial) pressure in a non-invasive way (or vice versa) requires determination of unknown distal-to-proximal (transfer) function which can be expressed by:

$$\Delta p = \sum_{i=1}^{n-1}\Delta p_i = \sum_{i=1}^{n-1} f\big(q_i(C_i, R_i, L_i)\big) \,, \tag{14}$$

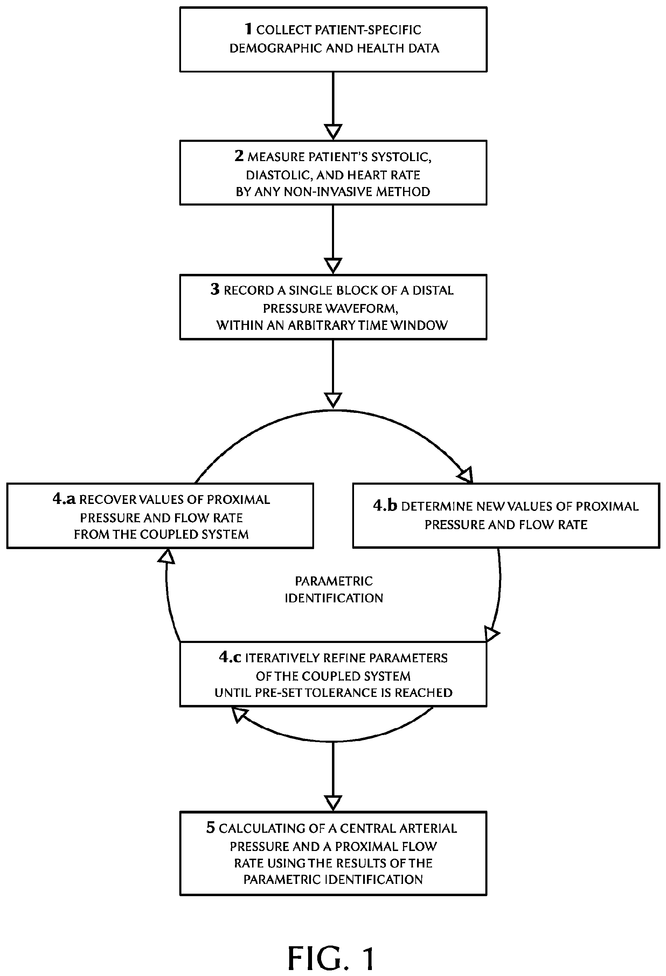

and which states that a pressure drop between selected locations is related to blood flow rates ($q_i$) which are expressed using vascular tree segments comprising compliance ($C_i$), resistance ($R_i$), and/or inertance ($L_i$). In a particular and the simplest embodiment (FIG. 3, 1-CRL) this is done using a 1-compartment CRL. In this embodiment, the distal-to-proximal transfer function has a form of a 1-equation relationship expressed by:

$$\Delta p = \left(L_1\frac{dq_0}{dt} + R_1 q_0 - L_1 C_0\frac{dp_0}{dt} - R_1 C_0\frac{dp_0}{dt}\right) \,. \tag{15}$$

In another embodiment: 2-equation relationship is used for a 2-compartment CRL (FIG. 3, 2-CRL). In another embodiment, a n-equation relationship is used for a n-compartment CRL (FIG. 3, n-CRL).

A practical implementation of distal-to-proximal transfer function in the form of (11), (12) and, in particular, (15), beside empirical patient-specific parameters such as $\{C_i, R_i, L_i\}$ requires precise knowledge about flow rates. Let's note: in case of n = 1 compartment model (1-CRL), the model must be completed by subsidiary $\{q_0\}$ flow relation, while for a n-

compartment - by $\{q_0, q_1, ..., q_{n-1}\}$ relations. In order to map distal and central pressures there is a need to have knowledge about a central compartment of circulation or, at least, knowledge about central flow rates. In some situations - also knowledge about further flow rates may be needed.

In a preferred embodiment of the present invention, said central $\{q_0\}$ or compartments $\{q_0, q_1, ..., q_{n-1}\}$ flow rates are expressed in terms of the adjoin lumped-parameter Windkessel model. Therefore - as opposed to many mentioned earlier attempts of looking for a transfer function - we do not assume structural rigidity of a transfer relationship, but we assume an evolutionary law that provides relationship that is needed to find the missing hemodynamical state quantities. It is one of the key ideas of the present invention - we abandon concepts that require treating a location of a distal measurement as part of the peripheral circulatory regions. A model of a central compartment only provides the missing relations for (11) and (12) or (15) but does not analyze a blood distribution to a peripheral region from the aorta. Note that, if we compute the missing flow rates separately and then use them to build a pressure wave transfer function, we will arrive at a fully uncoupled and segregated phenomenological models: a central compartment distributing to a peripheral, and the peripheral as such weekly (if at all) connected to the central.

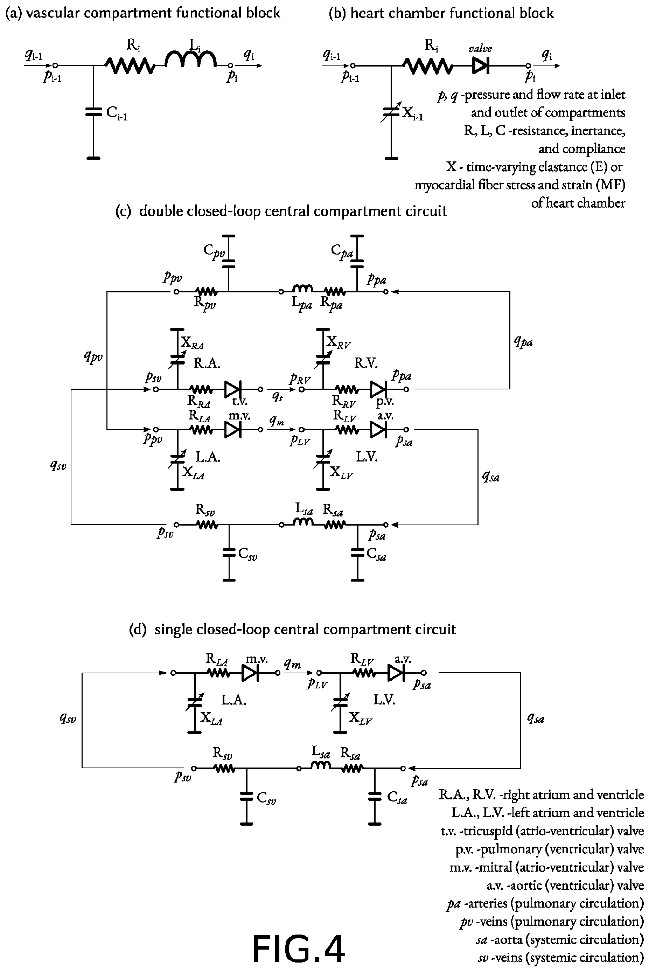

In a preferred embodiment of the present invention, a model of a central compartment of blood circulatory system is built using at least one lumped-parameter functional block CRL (presented on FIG. 4 (a)). Said lumped-parameter functional block CRL can comprise a valve which is a heart valve modeling diode. The diode should be understood as a model of a one-direction flow, in line with the terminology used in this field. The model can comprise of either a single closed-loop circuit FIG. 4 (d) or of two closed-loop circuits FIG. 4 (c). In these embodiments, a central compartment model contains at least two CRL functional blocks: the first representing large and middle-sized elastic vessels which exhibit significant inertio-elastic effects, and the second one that is representing resisto-capacitive effects. In a preferred embodiment of FIG. 4 (c) the model comprises a right heart circle analogy (as a superposition of two CRL building blocks: inertio-elastic determined by $C_{i-1}=C_{pa}$, $R_i=R_{pa}$, $L_i=L_{pa}$, and resisto-capacitive determined by $C_{i-1}=C_{pv}$, $R_i=R_{pv}$ and $L_i=0$) and a left heart circle analogy (again, inertio-elastic CRL block where $C_{i-1}=C_{sa}$, $R_i=R_{sa}$, $L_i=L_{sa}$ and resisto-capacitive with $C_{i-1}=C_{sv}$, $R_i=R_{sv}$ and $L_i=0$). In yet another preferred embodiment of the present invention, a central compartment model, with sufficient accuracy, can be built on the basis of a single closed-loop circuit of a systemic circulation (as depicted on FIG. 4 (d)), where an inertio-elastic CRL functional block is determined by $C_{i-1}=C_{sa}$, $R_i=R_{sa}$, $L_i=L_{sa}$, and resisto-capacitive is determined by $C_{i-1}=C_{sv}$, $R_i=R_{sv}$ and $L_i=0$). These embodiments of the invention are presented in diagrams (c) and (d) of FIG. 4, while the equations comprised in said embodiments obey equations (8) and (9) disclosed herein. The embodiment of FIG. 4 (c) is more precise in comparison to the one on FIG. 4 (d). However, the embodiment of FIG. 4 (d), while maintaining sufficient accuracy, is able to provide the results much faster when compared to the one on FIG. 4 (c). In further embodiments that provide an even more detailed description of a central compartment of blood circulatory system, $L_i$ is not equal to "0". In other embodiments, time-varying elastance concept (E) used in FIG. 4 (c) and FIG. 4 (d) is partially or totally replaced by myocardial fiber stress and strain concept (MF).

To reproduce physiological conditions, any closed-loop circuit as described herein of a central compartment preferably forms a self-excited oscillator. Therefore it is completed by a component that mimics hemodynamic action of a heart. A proper use of boundary conditions is an alternative to said self-excited oscillator. In order to incorporate said oscillator, the following considerations will apply. The internal anatomy of a heart reveals four chambers (left and right atria, and left and right ventricle) in the form of cavities enclosed by a fibrous wall endocardium, epicardium, and much more voluminous myocardium (as described in Barrett K et al. (2019) Ganong's Review of Medical Physiology, McGraw-Hill Education, Pappano AJ, Wier WG (2019) Cardiovascular Physiology, Elsevier, Klabunde RE (2018) Cardiovascular Physiology Concepts, Lippincott Williams & Wilkin). The two atria accept the blood returning to a heart from body's tissues and from lungs, respectively, while ventricles pumps blood to the lungs and all other organs. Each of the chambers is equipped with own valve that maintains the unidirectional flow. Each valve opens and closes (roughly) passively, according to blood pressure difference on each side of the valve. Therefore, a transvalvular flow and thereby a chamber outflow (according to the block presented on FIG. 4 (b)) can be expressed as:

$$q_i = \frac{\langle p_{i-1} - p_i \rangle}{R_i} , \qquad\qquad (16)$$

while a chamber volume can be expressed as:

$$-\frac{dV}{dt} = q_i - q_{i-1} . \qquad\qquad (17)$$

We could easily extend a chamber model as shown by numerous publications (e.g., Kim HJ et al. On coupling a lumped parameter heart model and a three-dimensional finite element aorta model. Annals of Biomedical Engineering. 2009;37(11):2153-69, Itu L, Sharma P, Suciu C. (2017) Patient-specific hemodynamic computations: application to personalized diagnosis of cardiovascular pathologies. Springer, Hongtao L. et al. (2020) A numerical model applied to the simulation of cardiovascular hemodynamics and operating condition of continuous-flow left ventricular assist device. J. Mathematical Biosciences and Engineering, 17(6): 7519-7543) to improve the quality of predictions. However, since hemodynamical activity of the individual chambers of a heart does not differ significantly - at approximately the same interval of time, pumps a similar volume of blood through the valve - any extension will not yield a significant improvement of quality. It is noted that since the characteristics of supplied flow into the right (pulmonary) and the left (systemic) circuits differ significantly, so does the pressures (see Caro CG et al. (2012) The Mechanics of the Circulation, Cambridge University Press). As the result, it is reasonable to look for a common representation for atrial and ventricular chambers pressure-volume characteristics.

In a preferred embodiment of the present invention, said heart chamber pressure-volume relation is formulated using the variable elastance concept (see Suga H. (1969) Time course of left ventricular pressure-volume relationship under various enddiastolic volume. Jpn Heart J. 1969;10(6):509-15, Walley KR (2016) Left ventricular function: time-varying elastance and left ventricular aortic coupling. Critical Care 20(270):1-11, Bozkurt S (2019) Mathematical modeling of cardiac function to evaluate clinical cases in adults and children. PloS One. 2019;14(10):e0224663, Li W (2020) Biomechanics of infarcted left ventricle: a review of modelling. Biomedical Engineering Letters. 10(3):387-417) which can be express by the following equation:

$$E(t_n) = E_{min} + E_n(t_n)(E_{max} - E_{min}), \qquad and\ t_n = \frac{t\%T}{t_{max}}, \qquad t_{max} = t@E(t) = E_{max}, \qquad (18)$$

therefore

$$p(t) = E(t)(V(t) - V_0). \qquad (19)$$

The variable elastance concept (E) is easier to implement and offers a good balance between the time needed to perform the method and accuracy of the results of the method.

In another embodiment of the present invention, the heart chamber pressure-volume closure relationship in a closed-loop lumped parameter central compartment model may be expressed directly in terms of the myocardial fiber stress and strain concept (MF, see Mirota K (2008) Constitutive Models of Vascular Tissue. Solid State Phenomena. Vol. 144, 100-105, Avazmohammadi R et al. A Contemporary Look at Biomechanical Models of Myocardium. Annual Review of Biomedical Engineering. 2019 Jun 4;21:417-442, Voigt JU, Cvijic M (2019) 2- and 3-Dimensional Myocardial Strain in Cardiac Health and Disease. JACC Cardiovasc Imaging. 12(9):1849-1863). In general, for all thin-walled and (near-) rotationally-symmetric geometries, the fiber stress ($\sigma_f$) changes are proportional to a bulk modulus $1/V \cdot dp/dV \sim \sigma_f$, and therefore - after integration - a relation for the cavity pressure ($p$) and fiber stress ($\sigma_f$) ratio is expressed by:

$$\frac{p}{\sigma_f} = \frac{1}{3}\ln\left(1 + \frac{V_w}{V}\right). \qquad (20)$$

Said heart chamber pressure-volume relationship, express by (19) or (20), completes the structure of the central compartment model and thus completes a model that provides a distal-to-proximal transition.

The myocardial fiber stress and strain concept (MF) is much more sophisticated when compared to the variable elastance concept (E). The reason is that it covers deformation and strain in a myocardial muscle fiber. This concept allows for a more accurate description which will yield, in some cases, even more accurate results.

Figure 1 depicts an embodiment of a method that is not claimed herein. The method of this embodiment comprises five steps: 1) collection of patient's general demographic and health data, 2) measurement of a patient's systolic, a patient's diastolic, and a patient's heart rate by a selected non-invasive method, 3) recording of a single block of a distal pressure waveform within a selected time window, 4) performing parametric identification of lumped-parameter models, including iterative refinement of the models until convergence is reached, and - finally - 5) determining of a central (proximal) pressure and a proximal flow with the use of the refined models. In other not illustrated on FIG. 1 embodiments that are claimed herein, said values of pressures and heart rate are assumed using a measured distal pressure waveform. The arbitrary time window is selected such that the method will include at least one full heart cycle. However, in extreme cases of an abnormally slow breathing rate (bradypnea) half of respiratory cycle length can be used. A skilled person will understand that such selection may be made using R waves. In preferred embodiments, the arbitrary time window will be selected such that the method will include two, three, four, five or more full heart cycles.

In the first step, patient's demographic and general medical data which are relevant for pressure pulse propagation in a patient's body are collected. While said data are only used in step 4), which is related to the parametric identification of the model, said data affect efficiency of the method. Efficiency can be measured by the time needed to arrive at the results of the method. In one embodiment, data relating to gender, age, weight and height are collected (following Smulyan H et al. (1998) Influence of body height on pulsatile arterial hemodynamic data. Journal of the American College of Cardiology. 31(5):1103-9, Christofaro DGD et al. (2017) Relationship between Resting Heart Rate, Blood Pressure and Pulse Pressure in Adolescents. Arquivos Brasileiros de Cardiologia. 108(5):405-410, Evans JM et al. (2017) Body Size Predicts Cardiac and Vascular Resistance Effects on Men's and Women's Blood Pressure. Front Physiol. 9;8:561, Gallo C et al. (2021) Testing a Patient-Specific In-Silico Model to Noninvasively Estimate Central Blood Pressure. Cardiovascular Engineering and Technology. 12(2):144-157). Drug therapies can also influence pulse wave propagation in a patient's body. In particular, influence can be observed for patient's taking drugs from the group of beta-adrenergic blocking agents (BBLOCK), angiotensin-converting-enzyme inhibitor (ACE), and/or antiarrhythmic agent (AARR) (see Harris WS, Schoenfeld CD, Weissler AM (1967) Effects of adrenergic receptor activation and blockade on the systolic preejection period, heart rate, and arterial pressure in man. Journal of Clinical Investigation. 46(11):1704-14, Morgan TO et al. (1974) A comparison of beta adrenergic blocking drugs in the treatment of hypertension. Postgraduate Medical Journal. 50(583):253-259, Nyberg G (1976) Effect of beta-adrenoreceptor blockers on heart rate and blood pressure in dynamic and isometric exercise. Drugs. 11 SUPPL 1:185-95, Fitzpatrick MA, Julius S (1985) Hemodynamic effects of angiotensin-converting enzyme inhibitors in essential hypertension: a review. Journal of Cardiovascular Pharmacology. 7 Suppl 1:S35-9, Ting CT et all (1993) Arterial hemodynamics in human hypertension. Effects of angiotensin converting enzyme inhibition. Hypertension. 22(6):839-46, Jobs A et al. (2019) Angiotensin-converting-enzyme inhibitors in hemodynamic congestion: a meta-analysis of early studies. Clinical Research in Cardiology. 108(11):1240-1248, Block PJ, Winkle RA (1983) Hemodynamic effects of antiarrhythmic drugs. American Journal of Cardiology. 52(6):14C-23C, Weiner B (1991) Hemodynamic effects of antidysrhythmic drugs. Journal of Cardiovascular Nursing. 5(4):39-48). In some embodiments, drug therapies are included in the method, in other embodiments drug therapies comprise the drugs listed above. Other drugs may also affect pulse wave propagation in a patient's body and, as the result, other embodiments comprise other drug therapies. It should be noted that drug therapies include also different dosing regimes as well as any further medical effects of drug therapies. Each of the above-mentioned factors can be used individually or combined in any fashion to obtain better starting values (initiating) for the model identification process for a particular patient's case. Better starting values directly affect efficiency of the method according to the invention. It is contemplated that in certain embodiments only selected patient-specific data are collected, e.g., gender, age or selected medications.

In the second step, in one embodiment that is claimed herein, a systolic blood pressure (SYS), a diastolic blood pressure (DIA), and a heart rate (HR) are assumed based on the measurements of a distal pressure waveform made in arbitrary units (AU). A skilled person has knowledge how to assume SYS, DIA and HR values from a measured distal pressure waveform. Main motivation for this approach is to obtain data that a) are from a source that is independent from a distal pressure waveform acquisition and b) that allow for calibration of the central compartment lumped-parameter model. This is important because it is generally assumed that distal pressure waveform measurements only provide information about waveform morphology. In this case it is acceptable that the result of a non-invasive measurement of a distal pressure waveform is expressed in arbitrary units and not necessarily in units of pressure. Thus, in one embodiment non-invasive measurements of distal pressure waveforms are made using arbitrary units (AU). Some of the devices which are used to record distal pressure waveforms have the ability to independently measure SYS, DIA and HR parameters - for example, if they are equipped with an upper arm cuff - then the result of such measurements will be used as a source of the previously mentioned data. It is therefore contemplated that in other embodiments, in the second step non-invasive measurements of distal pressure waveforms are made in units of pressure and no assumptions of values of SYS, DIA and HR are needed as SYS, DIA and HR are independently measured by the same or by a different device. In these embodiments, the measurements, including a distal pressure waveform, can be used directly in the calibration of the central compartment lumped-parameter model.

In other embodiments, the whole coupled system can be calibrated or only one of the models included in the coupled system is calibrated. In one specific embodiment, a distal-to-proximal transfer lumped-parameter model is calibrated using e.g., results of measurements from the radial artery, the aorta and using patient's demographic and general medical data.

In the third step, a non-invasive continuous recording of a distal pressure waveform is made. Most devices offering non-invasive measurement capabilities to scale the distal pressure waveform to values expressed in units of pressure (usually expressed in millimeters of mercury, denoted here mmHg or mm Hg), this is not necessary in the invention. The measured distal pressure can be expressed in arbitrary units (denoted here AU), because the morphology of the signal itself is crucial. Considering the limitations and preferences of clinical practice in measuring a continuous non-invasive blood pressure (CNBP), in a preferred embodiment of the present invention distal pressure measurements are assumed to be made in the radial artery. The preferred measurement methodology includes finger-cuff photoplethysmography and/or applanation tonometry. The recording and analysis of a distal pressure can be performed within a window of signal blocks covering a predetermined number of complete cycles corresponding to the systolic-diastolic action of a heart. In the preferred embodiment of the present invention, a window of width corresponding to the length of the respiratory cycle is analyzed (see Rodriguez-Molinero A (2013) Normal respiratory rate and peripheral blood oxygen saturation in the elderly population. Journal of the American Geriatrics Society. 61(12):2238-2240, Park C, Lee B (2014) Real-time estimation of respiratory rate from a photoplethysmogram using an adaptive lattice notch filter. Biomedical Engineering Online. 17;13:170, Scholkmann F, Wolf U (2019) The Pulse-Respiration Quotient: A Powerful but Untapped Parameter for Modern Studies About Human Physiology and Pathophysiology. Front Physiol. 9;10:371). In other embodiments that include extreme cases of an abnormally slow breathing rate (bradypnea), a half of respiratory cycle length is used.

In the fourth step, a model parametric identification process is performed. The structure of the model subject to parametric identification is defined by a coupled system that comprises two distinct models: a central compartment of blood circulatory system lumped-parameter model and a distal-to-proximal transfer lumped-parameter model. The whole model parametric identification process in this step is carried out in three substeps. First, a proximal pressure and a flow rate are calculated using a closed-loop central compartment of blood circulatory system lumped-parameter model. It should be emphasized that in the basic and preferred embodiment, the central compartment of blood circulatory system model comprises the systemic and pulmonary circulations (i.e., left and right heart cycles) for the most detailed and accurate results. However, the central compartment of blood circulatory system model can be restricted only to the systemic circulation without a significant loss of prediction accuracy. The values of the initial empirical parameters described earlier are provided with the use of patient demographic and general medical data gathered in the first step of the method. To be more precise, they are calculated from said patient demographic and general medical data and, if needed, with the use of literature references. Further, distal-to-proximal pressure approximations are determined according to the distal-to-proximal lumped-parameter model. Since both models that are forming the coupled system are analyzed as coupled, hence the error function is defined via the equation given below (where $\beta$ is the estimated model parameters set):

$$\left\| p_0 - \sum_{i=1}^{n-1} \Delta p_i - p_n \right\| = \ell(\beta) \leq \varepsilon(\beta). \tag{21}$$

In the third substep of the process, the error vector is calculated according to equation (21) in order to modify the values of the model's parameters in the next step and perform another iteration that seeks to minimize the error function. The literature on the subject provides a great number of useful methods for an efficient and effective solution of such a task (e.g., Walter E (1997) Identification of Parametric Models: from Experimental Data. Springer, Bock HG (2013) Model Based Parameter Estimation. Springer, Khoo M (2018) Physiological Control Systems: Analysis, Simulation, and Estimation John Wiley & Sons Bittanti S (2019) Model Identification and Data Analysis. John Wiley & Sons).

In a preferred embodiment of the present invention, the provision of the empirical parameters described above is formulated as an optimization task which can be based on minimization methods (see Villaverde AF et al. (2019) Benchmarking optimization methods for parameter estimation in large kinetic models. Bioinformatics. 35(5):830-838, Kreutz C (2019) Guidelines for benchmarking of optimization-based approaches for fitting mathematical models. Genome Biol.20(1):281, Schmiester L (2020) Efficient parameterization of large-scale dynamic models based on relative measurements. Bioinformatics. 36(2):594-602). Therefore, the error function (21) is used as the loss function of the optimization algorithm. This approach to providing the empirical parameters is much more flexible than known classical parametric identification approaches. Therefore, in a preferred embodiment of the present invention, the design of the method comprises a local search (minimization) algorithm above the mathematical structure of the coupled system. Its task is to directly control the process of selection of empirical parameter values in the fourth step of the method and to gradually improve the quality of a central arterial pressure prediction. In a preferred embodiment of the present invention, three local search (minimization) algorithms can be used as alternatives or in any combination. In one embodiment, a relatively stable and moderately complex method of Nelder-Mead can be used (see Nelder J, Mead R (1965) A simplex method for function minimization. Computer Journal, 7 (4): 308-313, Gao F, Han L (2010) Implementing the Nelder-Mead simplex algorithm with adaptive parameters. Computational Optimization and Applications, 51:1, 259-277). The method does not require computation of derivatives and uses only the values of the objective function as an n-dimensional simplex which is then geometrically transformed. In another embodiment, the local search (minimization) algorithm uses on of the Sequential Least Squares Programming methods (see Kraft D (1988) A software package for sequential quadratic programming. DFVLR, Braunschweig, Köln) and/or Broyden-Fletcher-Goldfarb-Shanno (see Zhu C, Byrd RH, Nocedal J (1997). Algorithm 778. L-BFGSB: Fortran routines for large scale bound constrained optimization. ACMTransactions on Mathematical Software, 23(4), 550-560). Each of those methods search the space determining the direction $\vec{p}$ based on solutions of the Newton-type equation $\nabla^2 f(x) \cdot \vec{p} = -\nabla f(x)$.

As to the general principle, the system of differential equations forming the circulation model creates a nonlinear self-exciting oscillator with a great sensitivity to initial conditions. Searching for a particular or a preferred solution under such circumstances is fraught with a considerable risk of failure. A much safer strategy, although less computationally efficient, is to perform initial identification of a certain set of solutions. Only then one can search for a solution that lies in the local minimum. Hence, in another embodiment of the present invention a local search is complemented by a global search e.g., in the form of the Adaptive Memory Programming for Global Optimization (AMPGO) and/ or the Simplicial Homology Global Optimization (SHGO). In the embodiment comprising AMPGO, at each step the locally given problem is solved by any of the previously mentioned methods (thus: Nelder-Mead, Sequential Least Squares Programming, or L-BFGSB). However, the local result is not directly used in the next step but is subject to a tunneling phase (see Lasdon L et al. (2010) Adaptive memory programming for constrained global optimization. Computers & Operations Research, 37(8):1500-1509). The AMPGO is extremely efficient and highly reliable. Its significant drawback is its large computational effort and consequently high computational power requirements. For AMPGO, a typical hardware platform might not provide an adequate power and thus can be limiting. In the preferred embodiment the Global algorithm is the SHGO (see Endres S (2017) A simplicial homology algorithm for Lipschitz optimization. Department of Chemical Engineering, University of Pretoria, Pretoria). Within this embodiment, a k-chain element coverage for the hypersurface of the objective function is constructed, and local tasks are solved with the use of a successive simplicial complex (see Mirota K (2008) Topological structure of finite element models of continuum mechanics. Bulletin of the Military University of Technology, LVII:2, 91-102).

Irrespectively of the used approach, the values of the empirical parameters of the coupled system can be iteratively refined against constant or altered values of the central arterial pressure and / or the proximal flow rate until convergence is reached. The term "iterative refinement" has an established meaning and a skilled person will understand what is covered by it. The same applies to the term "convergence". Convergence may be implemented, in one embodiment, as a constrain on variation of values of all or only selected empirical parameters. In other embodiments, convergence may be defined in terms of errors, e.g., mean absolute error. In another embodiment, convergence may be defined as a constrain on variation of values of the central arterial pressure and / or the proximal flow rate or any parametrical representation thereof. Various numerical and statistical tests known for a skilled person can be used to determine whether a convergence is reached.

Last, fifth step, is used to calculate values of a central arterial pressure and a proximal flow rate based on the results of the parametric identification step. For the avoidance of doubts, the results of the parametric identification step comprise the coupled system with the empirical parameters fine tuned for a specific patient. The calculated values with the method can be outputted in any suitable way and form. The knowledge about said proximal (central) arterial pressure and said proximal flow rate in a given time window allows for reconstruction of a central arterial pressure waveform morphology. This is useful in diagnosis and treatment of an elevated blood pressure and / or hypertension.

The various embodiments of the method of the invention were evaluated in detail. Below there are results of validation of the embodiment implementing a model of FIG 4 (c) with X = E. The method of the invention was validated based on medical experiment results from a multi-center, non-randomized clinical trial. Patients' demographic and health data as well as results of measurements are summarized in Table 1 below (the measured values are expressed as a mean $\pm$ standard deviation).

| Table 1. Baseline characteristics of the clinical experiment population | | | |
|---|---|---|---|
| Parameter | Female | Male | All cases |
| Number of cases | 30 (54.5%) | 25 (45.5%) | 55 (100%) |
| Myocardial ischemia | 8 (26.7%) | 17 (68.0%) | 25 (45.5%) |
| Age [years] | 68.57±8.49 | 67.12±8.96 | 67.91±8.65 |
| Height [cm] | 161.53±5.67 | 174.24±7.52 | 167.31±9.12 |
| Weight [kg] | 72.00±11.33 | 88.52±14.09 | 79.51±15.03 |
| HR [1/min] | 68.30±5.06 | 68.72±6.02 | 68.49±5.47 |
| SYS [mmHg] | 131.83±11.03 | 136.72±13.46 | 134.05±12.32 |
| DIA [mmHg] | 79.47±9.93 | 82.56±8.09 | 80.87±9.19 |
| HCT [ml/100 ml] | 40.43±3.12 | 42.20±4.45 | 41.24±3.85 |
| RBC [$10^3/\mu$l] | 4.41±0.42 | 4.58±0.60 | 4.49±0.51 |
| HGB [g/dl] | 13.49±1.10 | 14.24±1.69 | 13.83±1.44 |
| PLT [$10^3/\mu$l] | 241.17±59.69 | 201.56±50.95 | 223.16±58.85 |
| BBLOCK | 21 (70.0%) | 17 (68.0%) | 38 (69.1%) |
| ACE | 7 (23.3%) | 12 (48%) | 19 (34.5%) |

(continued)

| Table 1. Baseline characteristics of the clinical experiment population | | | |
|---|---|---|---|
| Parameter | Female | Male | All cases |
| AARR | 22 (73.3%) | 17 (68%) | 39 (70.9%) |
| HR-heart rate, SYS and DIA-systolic and diastolic pressure, HCT-hematocrit, RBC,HGB,PLT-red blood cell, hemoglobin, platelets count, BBLOCK -beta-adrenergic blocking agent, ACE-angiotensin-converting-enzyme inhibitor, AARR -antiarrhythmic agent | | | |

Generally, in this study all admitted patients had clinical indications of a coronary artery disease. For each patient, continuous non-invasive blood pressure measurements (CNBP) were performed on the radial artery with a sampling rate of 100 Hz. In addition, an independent non-invasive measurement was performed in the brachial artery using the oscillometric method.

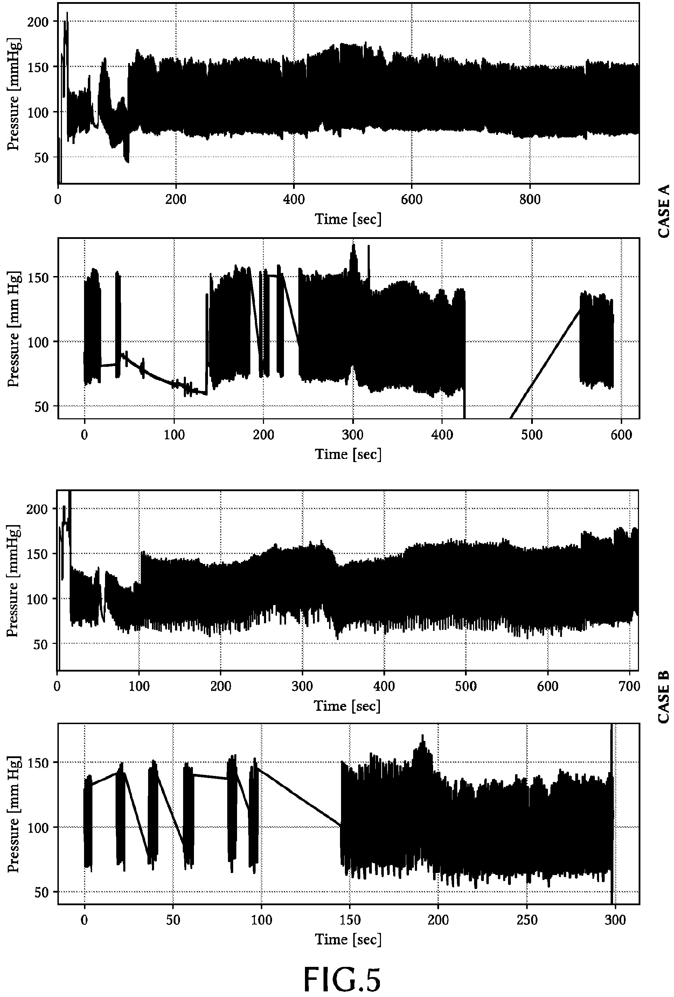

Since the patients underwent invasive diagnostics, the results of intra-aortic catheter pressure measurements were used as reference data. The pressure signal obtained using invasive measurements was sampled at 200 Hz. As an example, FIG. 5 shows complete pressure recordings for two patients (labeled CASE A and CASE B), with non-invasive (made using a finger cuff photoplethysmography technique) recordings at the top and invasive (made using an intraarterial catheter) recordings at the bottom.

As the records pertaining to the CASE B show, there are obvious signs of cardiac arrhythmias (see irregularities in the heartbeat and dysrhythmia). According to the clinical case report form (CRF), the patient was treated with AARR (antiarrhythmic) drugs.

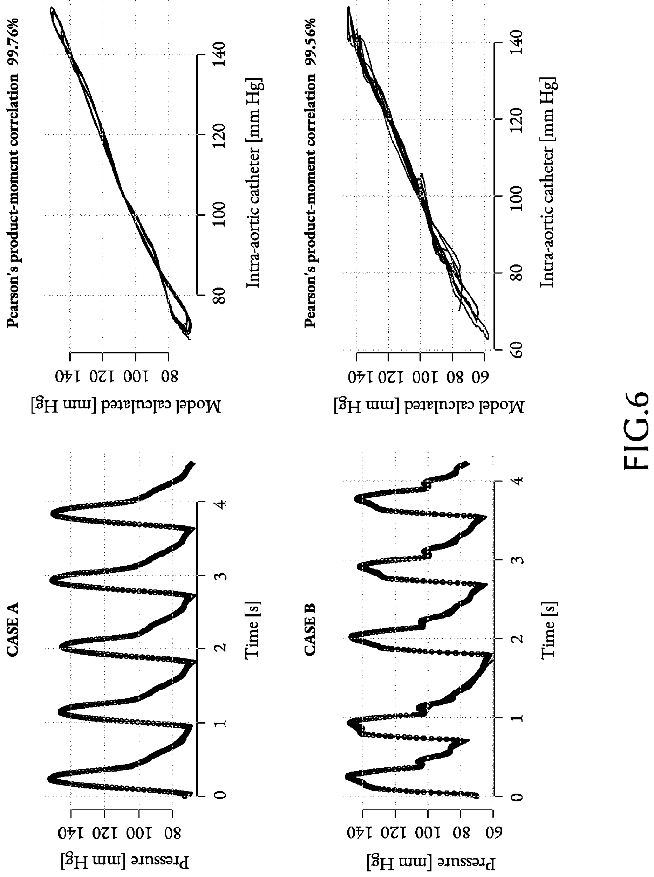

FIG. 6 shows the results of a central arterial pressure reconstruction with respect to reference data for the two cases presented in FIG. 5. While the reconstruction result is fully satisfactory for both cases, the negative effect of CASE B arrhythmia is clearly visible.

Reconstruction of a central arterial pressure can be made using either local or global minimization algorithms or a combination of both types of minimization algorithms.

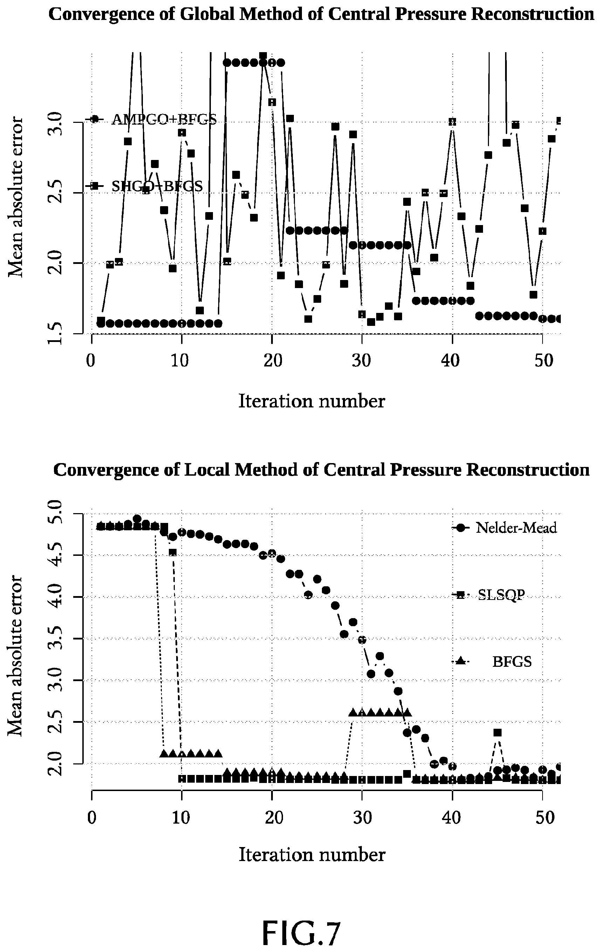

The preferred embodiment of the central arterial pressure reconstruction method is based on a local minimization algorithm including, for example, Nelder-Mead, SLAQP and/or L-BFGSB. In FIG. 7, one can see the convergence of the minimization process achieved using Nelder-Mead, SLAQP, or L-BFGSB (patient with arrythmia, CASE B). It is evident that each local minimization algorithm successfully accomplished the reconstruction task. The error measured as MAE (mean absolute error) was respectively: 0.1196667, 0.1203333 and 0.1203333. Similarly, calculation of RMSE (root-mean-square error) gave: 0.1401587, 0.1407667 and 0.1408015. However, the nature of convergence was quite different. In the case of the Nelder-Mead algorithm, the iterative process converged fairly stably but very slowly. In contrast, SLAQP and L-BFGSB converged much faster, although it required additional effort to damp numerical oscillations and to achieve convergence.

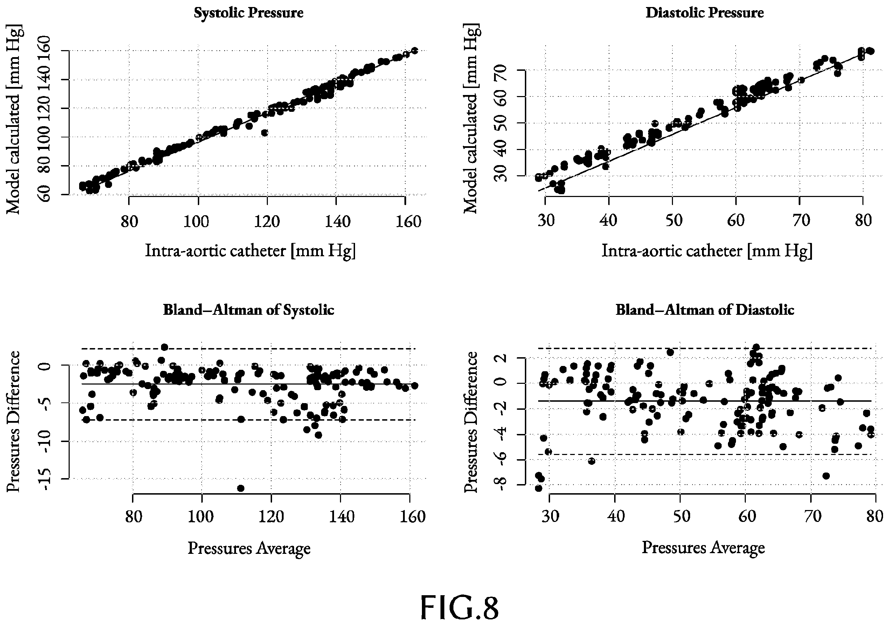

Alternatively or additionally, one or more global minimization algorithms can be used. The bottom part of FIG. 7 shows the convergence results using the two global algorithms AMPGO and SHGO. The final summary of the results obtained with the method of present invention implementing global algorithms is shown in FIG. 8. The figure summarizes the values of systolic and diastolic pressures (left and right sides of the figure, respectively), obtained from invasive measurements and from calculations implementing the method of the present invention. The upper part of the figure contains correlation plots and the lower part the Bland-Altman plot (Tukey mean-difference plot) for all cases comprised in the clinical trial presented in a window comprising five cycles. More than 250 validation cases presented in FIG. 8 clearly shows a very good agreement between the results obtained invasively and the results obtained with the use of the method according to the invention. It should be noted that other one or more global minimization algorithms can be used in the method with or alternatively to AMPGO and/or SHGO algorithms.

In another embodiments, various combinations of local and global minimization algorithms are used to arrive at e.g., systolic and diastolic pressures. Said various combinations are used to meet specific implementation needs. In said embodiments one or more local minimization algorithms (for example, Nelder-Mead, SLAQP, and/or L-BFGSB) can be used before, after and/or as layers with one or more global minimization algorithms (for example, AMPGO and/or SHGO. In some embodiments, only one or more local minimization algorithm can be used. Since said algorithms are less computational demanding, they can arrive at results faster or embodiments comprising only said algorithms can be implemented on portable devices which can have a lower computational power. In other embodiments, a portable device is used in communication with a server configured to make all demanding computations. Said communication can use a local network or Internet.

Any calculation step or substep of the method according to the invention can be implemented using a computer or a computer program. In some specific embodiments, some or all calculations are done using a computer program stored on a computer or on any type of a memory device or both. In another embodiments, some or all calculations for the purpose of the method can be done remotely e.g., using cloud-based infrastructure which can include the use of Internet or a local network.

While all measurement methods and minimization algorithms described here are intended to define the parameters of the invention and to provide tangible results to be compared with clinical trials, there are by no means limiting and are exemplary. The words like "including" or "comprising" are not limiting and, for example, when an element A includes another element B, the element A may include other element or elements in addition to the element B. The use of a singular or plural form is not limiting for the scope of the disclosure and, for example, a part of the description which is indicating that an element A contains an element B, this part also discloses that multiple elements B are contained in one element A and multiple elements A are contained in one element B as well as an embodiment where multiple elements A contain multiple elements B. Many other embodiments will be apparent and clear for those skilled in the art upon reviewing the content of the present disclosure. The scope of the invention is determined by the appended claims.

## Claims

1. A method of reconstruction of a central arterial pressure waveform morphology for a human patient from a distal non-invasive continuous pressure measurement, wherein the method comprises the steps of:

   - performing parametric identification (4) of a coupled system using patient-specific demographic and health data and using assumed patient's systolic pressure, patient's diastolic pressure and patient's heart rate, wherein the patient-specific demographic and health data affect pressure pulse propagation in a patient's body, wherein the coupled system comprises a central compartment of blood circulatory system lumped-parameter model and a distal-to-proximal transfer lumped-parameter model, wherein the assumption of the patient's systolic pressure, patient's diastolic pressure and patient's heart rate is done using a registered patient's distal pressure waveform, wherein said registration of the patient's distal pressure waveform is done continuously and within a time window that comprises at least one entire cycle of patient's heart or half of one entire patient's respiratory cycle and wherein the registration is done non-invasively; and
   - calculating (5) of a central arterial pressure and a proximal flow rate using the results of the parametric identification.

2. The method of claim 1, wherein the patient-specific demographic and health data include gender, age, body height, general fitness assessment and/or current medication, wherein the current medication includes, but is not limited to, a beta-adrenergic blocking agent, an angiotensin-converting-enzyme inhibitor and/or an antiarrhythmic agent.

3. The method of any of claims 1-2, wherein the continuous registration of the pressure waveform is made from the distal artery and/or comprises measuring an arterial blood pressure by a sensor positioned above the radial artery using methods selected from photoplethysmography and/or applanation tonometry.

4. The method of any of claims 1-3, wherein the time window comprises a sequence of consecutive full cycles of a heart that are within a single respiratory cycle, wherein the sequence comprises two, three, four, five or more consecutive cycles of the patient's heart that are within said single respiratory cycle.

5. The method of any of claims 1-4, wherein the central compartment model comprises at least one lumped-parameter functional block (CRL), wherein said lumped-parameter functional block (CRL) is selected from a group that comprises a vascular compartment functional block (CRL) and a heart chamber functional block, wherein said vascular compartment functional block (CRL) has the following structure:

wherein

$C_i$ is compliance, $R_i$ is resistance, $L_i$ is inertance, $q_i$ is flow rate, $p_i$ is pressure, and i is compartment number, and wherein said heart chamber functional block has the following structure:

wherein:

X is time-varying elastance concept (E) or myocardial fiber stress and strain concept (MF), $R_i$ is resistance, *valve* is heart valve modeling diode, $q_i$ is flow rate, $p_i$ is pressure, and i is compartment number.

6. The method of claim 5, wherein the central compartment model comprises at least two said functional blocks (CRL), wherein the first one is representing large and middle-sized elastic vessels which exhibit significant inertio-elastic effects, and the second one is representing resisto-capacitive effects.

7. The method of claim 6, wherein the central compartment model comprises a right heart circle in the form of a superposition of two said functional blocks (CRL), wherein the inertio-elastic functional block is determined by $C_{i-1}=C_{pa}$, $R_i=R_{pa}$, $L_i=L_{pa}$, and the resisto-capacitive functional block is determined by $C_{i-1}=C_{pv}$, $R_i=R_{pv}$ and $L_i=0$.

8. The method of any of claims 5-7, wherein the central compartment model comprises the following structure:

wherein:

$p$ is pressure, $q$ is flow rate, R is resistance, $L$ is inertance, $C$ is compliance, $X$ is time-varying elastance concept (E) or myocardial fiber stress and strain concept (MF), *R.A., R.V.* is right atrium and ventricle, *L.A., L.V.* is left atrium and ventricle, *t.v.* is tricuspid (atrio-ventricular) valve, *p.v.* is pulmonary (ventricular) valve, *m.v.* is mitral (atrio-ventricular) valve, *a.v.* is aortic (ventricular) valve, *pa* are arteries (pulmonary circulation), pv are veins (pulmonary circulation), *sa* is aorta (systemic circulation), and sv are veins (systemic circulation).

9. The method of any of claims 5-6, wherein the central compartment model comprises a single closed-loop circuit of a systemic circulation where the inertio-elastic functional block is determined by $C_{i-1}=C_{sa}$, $R_i=R_{sa}$, $L_i=L_{sa}$, and the

resisto-capacitive functional block is determined by $C_{i-1}=C_{sv}$, $R_i=R_{sv}$ and $L_i=0$.

**10.** The method of any of claims 5-6 or 9, wherein the central compartment model comprises the following structure:

wherein:

$p$ is pressure, $q$ is flow rate, R is resistance, $L$ is inertance, $C$ is compliance, $X$ is time-varying elastance concept (E) or myocardial fiber stress and strain concept (MF), *L.A., L.V.* is left atrium and ventricle, m.v. is mitral (atrio-ventricular) valve, *a.v.* is aortic (ventricular) valve, *pv* are veins (pulmonary circulation), *sa* is aorta (systemic circulation), and sv are veins (systemic circulation).

**11.** The method of any of claims 5-10, wherein the central compartment model comprises the following conditions:

$$q_i = \frac{(p_{i-1}-p_i)}{R_i} \text{ and } -\frac{dV}{dt} = q_i - q_{i-1}.$$

**12.** The method of any of claims 5-10, wherein the central compartment model comprises a chamber pressure-volume relation formulated using the variable elastance concept (E).

**13.** The method of any of claims 5-10, wherein the central compartment model comprises a chamber pressure-volume relation formulated using the myocardial fiber stress and strain concept (MF).

**14.** The method of any of claims 1-13, wherein the parametric identification (4) of the coupled system comprises:

- calculating initial values of empirical parameters of the coupled system using said patient's demographic and health data;
- solving equations of the coupled system using the initial values of empirical parameters to calculate an approximated value of the central arterial pressure and / or the proximal flow rate in a selected time window; and
- iteratively refining (4.c) the values of the empirical parameters of the coupled system against constant or altered values of the central arterial pressure and / or the proximal flow rate until convergence is reached.

**15.** The method of claim 14, wherein said calculating of initial values of empirical parameters of the coupled system is done only for the central compartment of blood circulatory system lumped-parameter model.

**16.** The method of any of claims 14-15, wherein said empirical parameters comprise compliance, resistance and/or inertance and/or wherein said empirical parameters comprise parameters of the time-varying elastance concept or parameters of the myocardial fiber stress and strain concept.

**17.** The method of any of claims 14-16, wherein said iterative refinement comprises at least one minimization algorithm, wherein each said minimization algorithm is selected from a group that consists of a local minimization algorithm and a global minimization algorithm.

**18.** The method of any of claims 17, wherein the local minimization algorithm is from a group that consists of the Nelder-Mead, the Sequential Least Squares Programming and the Broyden-Fletcher-Goldfarb-Shanno.

**19.** The method of any of claims 16-18, wherein the global minimization algorithm is selected from a group that consists of the Adaptive Memory Programming for Global Optimization and the Simplicial Homology Global Optimization.

20. The method of any of claims 16-19, wherein said iterative refinement comprises a step of refinement that implements the global minimization algorithm followed by a step of refinement that implements the local minimization algorithm.

21. A computer-readable [storage] medium comprising instructions which, when executed by a computer, cause the computer to carry the steps of a method defined in any of claims 1-20.

22. A system for reconstruction of a central arterial pressure waveform morphology from a distal non-invasive continuous pressure measurement, wherein the system comprises:

- a registering means for non-invasive continuous registration of a pressure waveform from a human patient;
- a measuring means for non-invasively measuring of a patient's systolic pressure, a patient's diastolic pressure, and a patient's heart rate; and
- a computer adapted to perform the steps of the method defined in any of claims 1-20.

**Patentansprüche**

1. Das Verfahren zur Rekonstruktion einer zentralen Arteriendruckwellenformmorphologie für einen menschlichen Patienten aus einer distalen nicht-invasiven kontinuierlichen Druckmessung, wobei das Verfahren die folgenden Schritte umfasst:

- Durchführen parametrischer Identifizierung (4) eines gekoppelten Systems unter Verwendung patientenspezifischer demografischer und gesundheitlicher Daten und unter Verwendung des angenommenen systolischen Drucks des Patienten, des diastolischen Drucks des Patienten und der Herzfrequenz des Patienten, wobei die patientenspezifischen demografischen und gesundheitlichen Daten die Druckpulsausbreitung im Körper eines Patienten beeinflussen, wobei das gekoppelte System einen zentralen Kammer des Lumped-Parameter-Modells des Blutkreislaufsystems und ein Lumped-Parameter-Modell für den Transfer von distal nach proximal umfasst, wobei die Annahme des systolischen Drucks des Patienten, des diastolischen Drucks des Patienten und der Herzfrequenz des Patienten unter Verwendung der distalen Druckwellenform eines registrierten Patienten erfolgt, wobei die Registrierung der distalen Druckwellenform des Patienten kontinuierlich und innerhalb eines Zeitfensters erfolgt, das mindestens einen gesamten Zyklus des Herzens des Patienten oder die Hälfte des gesamten Atemzyklus des Patienten umfasst, und wobei die Registrierung nicht-invasiv erfolgt, und
- Berechnen (5) eines zentralen Arteriendrucks und einer proximalen Flussrate unter Verwendung der Ergebnisse der parametrischen Identifizierung.

2. Das Verfahren nach Anspruch 1, wobei die patientenspezifischen demografischen und gesundheitlichen Daten Geschlecht, Alter, Körpergröße, allgemeine Fitnessbewertung und/oder aktuelles Medikament beinhalten, wobei das aktuelle Medikament unter anderem ein beta-adrenerges Blockierungsmittel, einen Angiotensin-Converting-Enzym-Inhibitor und/oder ein Antiarrhythmikum beinhaltet.

3. Das Verfahren nach einem der Ansprüche 1-2, wobei die kontinuierliche Registrierung der Druckwellenform von der distalen Arterie vorgenommen wird und/oder das Messen eines arteriellen Blutdrucks durch einen Sensor, der über der radialen Arterie positioniert ist, unter Verwendung von Verfahren, die aus Photoplethysmographie und/oder Applanationstonometrie ausgewählt sind, umfasst.

4. Das Verfahren nach einem der Ansprüche 1-3, wobei das Zeitfenster eine Sequenz aufeinanderfolgender vollständiger Zyklen eines Herzens umfasst, die innerhalb eines einzelnen Atmungszyklus liegen, wobei die Sequenz zwei, drei, vier, fünf oder mehr aufeinanderfolgende Zyklen des Herzens des Patienten umfasst, die innerhalb des einzelnen Atmungszyklus liegen.

5. Das Verfahren nach einem der Ansprüche 1-4, wobei das zentrale Kammermodell mindestens einen Lumped-Parameter-Funktionsblock (CRL) umfasst, wobei der Lumped-Parameter-Funktionsblock (CRL) aus einer Gruppe ausgewählt ist, die einen Gefäßkammer-Funktionsblock (CRL) und einen Herzkammer-Funktionsblock umfasst, wobei der Gefäßkammer-Funktionsblock (CRL) die folgende Struktur aufweist:

wobei

$C_i$ Nachgiebigkeit ist, $R_i$ Widerstand ist, $L_i$ Trägheit ist, $q_i$ Durchfluss ist, $p_i$ Druck ist und i Kammernummer ist,

und wobei der Herzkammer-Funktionsblock die folgende Struktur beinhaltet:

wobei:

$X$ das zeitvariable Elastanzkonzept (E) oder das Myokardfaser-Stress- und Dehnungskonzept (MF) ist, $R_i$ Widerstand ist, *das Ventil* Herzklappenmodellierungsdiode ist, $q_i$ Durchflussrate ist, $p_i$ Druck ist und i Kammernummer ist.

6. Das Verfahren nach Anspruch 5, wobei das zentrale Kammermodell mindestens zwei der Funktionsblöcke (CRL) umfasst, wobei der erste große und mittelgroße elastische Gefäße darstellt, die signifikante inertio-elastische Effekte aufweisen, und der zweite resisto-kapazitive Effekte darstellt.

7. Das Verfahren nach Anspruch 6, wobei das zentrale Kammermodell einen rechten Herzkreis in Form einer Überlagerung von zwei der Funktionsblöcke (CRL) umfasst, wobei der inerte-elastische Funktionsblock durch $C_-=C_{pa}$, $R_i=R_{pa}$, $L_i=L_{pa}$ und der resisto-kapazitive Funktionsblock durch $C_{i-1}=C_{pv}$, $R_i=R_{pv}$ und $L_i=0$ bestimmt wird.

8. Das Verfahren nach einem der Ansprüche 5-7, wobei das zentrale Kammermodell die folgende Struktur umfasst:

wobei:

$p$ Druck ist, $q$ Flussrate ist, R Widerstand ist, $L$ Trägheit ist, C Nachgiebigkeit ist, $X$ zeitvariables Elastanzkonzept (E) ist oder Myokardfaser-Stress- und Dehnungskonzept (MF), *R.A., R.V.* rechtes Atrium und rechter Ventrikel ist, *L.A., L.V.* linkes Atrium und linker Ventrikel ist, *t.v.* (atrio-ventrikuläre) Trikuspidalklappe ist, p.v. (ventrikuläre) Pulmonalklappe ist, m.v. (atrio-ventrikuläre) Mitralklappe ist, a.v. (ventrikuläre) Aortenklappe ist, *pa* Arterien (pulmonaler Kreislauf) sind, pv Venen (pulmonaler Kreislauf) sind, *sa* Aorta (systemischer Kreislauf) ist und sv Venen (systemischer Kreislauf) sind.

**9.** Das Verfahren nach einem der Ansprüche 5-6, wobei das zentrale Kammermodell einen einzelnen geschlossenen Kreislauf eines Körperkreislaufs umfasst, wobei der inertioelastische Funktionsblock durch $C_{i-1}=C_{sa}$, $R_i=R_{sa}$, $L_i=L_{sa}$ und der resisto-kapazitive Funktionsblock durch $C_{i-1}=C_{sv}$, $R_i=R_{sv}$ und $L_i=0$ bestimmt wird.

**10.** Das Verfahren nach einem der Ansprüche 5-6 oder 9, wobei das zentrale Kammermodell die folgende Struktur umfasst:

wobei:

$p$ Druck ist, $q$ Flussrate ist, R Widerstand ist, $L$ Trägheit ist, C Nachgiebigkeit ist, $X$ zeitvariables Elastanzkonzept (E) oder Myokardfaser-Stress- und Dehnungskonzept (MF) ist, *L.A., L.V.* linkes Atrium und linker Ventrikel ist, m.v. (atrio-ventrikuläre) Mitralklappe ist, a.v. (ventrikuläre) Aortenklappe ist, pv Venen (Lungenkreislauf) sind, *sa* Aorta (Körperkreislauf) ist und sv Venen (Körperkreislauf) sind.

**11.** Das Verfahren nach einem der Ansprüche 5-10, wobei das zentrale Kammermodell die folgenden Bedingungen umfasst:

$$q_i = \frac{\langle p_{i-1} - p_i \rangle}{R_i} \text{ und } -\frac{dV}{dt} = q_i - q_{i-1}.$$

**12.** Das Verfahren nach einem der Ansprüche 5-10, wobei das zentrale Kammermodell eine Kammerdruck-Volumen-Beziehung umfasst, die unter Verwendung des variablen Elastanzkonzepts (E) formuliert wurde.

**13.** Das Verfahren nach einem der Ansprüche 5-10, wobei das zentrale Kammermodell eine Kammerdruck-Volumen-Beziehung umfasst, die unter Verwendung des Myokardfaser-Stress- und Dehnungskonzepts (MF) formuliert wurde.

**14.** Das Verfahren nach einem der Ansprüche 1-13, wobei die parametrische Identifizierung (4) des gekoppelten Systems Folgendes umfasst:

- Berechnen von Anfangswerten empirischer Parameter des gekoppelten Systems unter Verwendung der demografischen und gesundheitlichen Daten des Patienten;
- Lösen von Gleichungen des gekoppelten Systems unter Verwendung der Anfangswerte empirischer Parameter, um einen ungefähren Wert des zentralen Arteriendrucks und / oder der proximalen Flussrate in einem ausgewählten Zeitfenster zu berechnen; und
- iterative Verfeinerung (4.c) der Werte der empirischen Parameter des gekoppelten Systems gegen konstante oder veränderte Werte des zentralen Arteriendrucks und / oder der proximalen Flussrate, bis Konvergenz erreicht ist.

**15.** Das Verfahren nach Anspruch 14, wobei das Berechnen von Anfangswerten von empirischen Parametern des gekoppelten Systems nur für das zentrale Kammer des Lumped-Parameter-Modells des Blutkreislaufsystems durchgeführt wird.

**16.** Das Verfahren nach einem der Ansprüche 14-15, wobei die empirischen Parameter Nachgiebigkeit, Widerstand und/oder Trägheit umfassen und/oder wobei die empirischen Parameter Parameter des zeitlich variierenden Elastanzkonzepts oder Parameter des Myokardfaser-Stress- und Dehnungskonzepts umfassen.

**17.** Das Verfahren nach einem der Ansprüche 14-16, wobei die iterative Verfeinerung mindestens einen Minimierungsalgorithmus umfasst, wobei jeder der Minimierungsalgorithmen aus einer Gruppe ausgewählt ist, die aus einem lokalen Minimierungsalgorithmus und einem globalen Minimierungsalgorithmus besteht.

**18.** Das Verfahren nach einem der Ansprüche 17, wobei der lokale Minimierungsalgorithmus aus einer Gruppe stammt, die aus dem Nelder-Mead, der Sequential Least Squares Programming und dem Broyden-Fletcher-Goldfarb-Shanno besteht.

**19.** Das Verfahren nach einem der Ansprüche 16-18, wobei der globale Minimierungsalgorithmus aus einer Gruppe ausgewählt ist, die aus der adaptiven Speicherprogrammierung für die globale Optimierung und der globalen Simplicial-Homology-Optimierung besteht.

**20.** Das Verfahren nach einem der Ansprüche 16-19, wobei die iterative Verfeinerung einen Verfeinerungsschritt umfasst, der den globalen Minimierungsalgorithmus implementiert, gefolgt von einem Verfeinerungsschritt, der den lokalen Minimierungsalgorithmus implementiert.

**21.** Ein computerlesbares [Speicher] Medium, das Anweisungen umfasst, die, wenn sie von einem Computer ausgeführt werden, den Computer veranlassen, die Schritte eines in einem der Ansprüche 1-20 definierten Verfahrens durchzuführen.

**22.** Ein System zur Rekonstruktion einer zentralen Arteriendruckwellenformmorphologie aus einer distalen nicht-invasiven kontinuierlichen Druckmessung, wobei das System Folgendes umfasst:

- ein Registrierungsmittel zur nicht-invasiven kontinuierlichen Registrierung einer Druckwellenform von einem menschlichen Patienten;
- eine Messeinrichtung zum nicht-invasiven Messen des systolischen Drucks eines Patienten, des diastolischen Drucks eines Patienten und der Herzfrequenz eines Patienten; und
- einen Computer, der dafür geeignet ist, die Schritte des in einem der Ansprüche 1-20 definierten Verfahrens durchzuführen.

**Revendications**

1. Procédé pour reconstruire d'une morphologie de forme d'onde de la pression artérielle centrale pour un patient humain à partir d'une mesure distale continue non invasive de la pression, le procédé comprenant des étapes de :

   - effectuer d'identification paramétrique (4) d'un système couplé en utilisant des données de santé et des données démographiques spécifiques au patient et en utilisant la pression systolique présumée, la pression diastolique présumée et la fréquence cardiaque présumée du patient, données de santé et données démographiques spécifiques au patient affectant la propagation d'onde de pouls dans un corps du patient, le système couplé comprenant un compartiment central du modèle de paramètre groupé du système de circulation sanguine un modèle de paramètre groupé du transfert distal-proximal, la présomption de pression systolique du patient, de pression diastolique du patient et de fréquence cardiaque du patient est réalisée en utilisant de forme d'onde de pressure distale du patient enregistrée, ledit enregistrement de forme d'onde de pressure distale du patient est réalisé en continu et dans une fenêtre temporelle qui comprend au moins un cycle entier du cœur du patient ou la moitié d'un cycle respiratoire entier et l'enregistrement est réalisé de manière non invasive ; et
   - calculer (5) une pression artérielle centrale et un débit sanguin proximale en utilisant des résultats d'identification paramétrique.

2. Procédé selon la revendication 1, des données de santé et démographiques spécifiques au patient incluant le sexe, l'âge, la taille, l'évaluation de condition physique générale et/ou la médication actuelle, la médication actuelle incluant sans s'y limiter un bêta-bloquant, un inhibiteur de l'enzyme de conversion de l'angiotensine et/ou un agent antiarythmique.

3. Procédé selon l'une quelconque des revendications 1-2, l'enregistrement en continu de forme d'onde de pression étant fait à partir d'artère distale et/ou comprenant mesurage de pression artérielle par un capteur positionné au-dessus de l'artère radiale en utilisant des méthodes choisies parmi photopléthysmographie et/ou tonométrie à aplanation.

4. Procédé selon l'une quelconque des revendications 1-3, la fenêtre temporelle comprenant une séquence des cycles complets consécutifs d'un cœur qui sont dans un cycle respiratoire unique, la séquence comprenant deux, trois, quatre, cinq ou plusieurs cycles consécutifs du cœur du patient qui sont dans ledit cycle respiratoire unique.

5. Procédé selon l'une quelconque des revendications 1-4, le modèle du compartiment central comprenant au moins un bloc fonctionnel de paramètre groupé (CRL), ledit bloc fonctionnel de paramètre groupé (CRL) est choisi dans le groupe qui comporte un bloc fonctionnel de compartiment vasculaire (CRL) et un block fonctionnel de chambre cardiaque, ledit bloc fonctionnel de compartiment vasculaire (CRL) ayant la structure suivante :

   dans laquelle
   $C_i$ est la compliance, $R_i$ est la résistance, $L_i$ est l'inertance, $q_i$ est le débit, $p_i$ est la pression, et i est un nombre du compartiment,

   et ledit block fonctionnel de chambre cardiaque ayant la structure suivante :

dans laquelle :
$X$ est la notion d'élastance variant dans le temps (E) ou de contrainte et de déformation des fibres myocardiques (MF), $R_i$ est la résistance, *valve* est une diode modélisant une valve cardiaque, $q_i$ est le débit, $p_i$ est la pression, et *i* est un nombre du compartiment.

6. Procédé selon la revendication 5, le modèle du compartiment central comprenant au moins deux blocs fonctionnels (CRL), le premier représentant grands et moyens vaisseaux élastiques qui présentent des effets inertio-élastiques importantes, et le second représentant des effets résisto-capacitatifs.

7. Procédé selon la revendication 6, le modèle du compartiment central comprenant un cycle du cœur droit en forme d'une superposition de deux lesdits blocs fonctionnels (CRL), le bloc fonctionnel inertio-élastique étant déterminé par $C_{i-1}=C_{pa}$, $R_i=R_{pa}$, $L_i=L_{pa}$, et le bloc fonctionnel résisto-capacitatif étant déterminé par $C_{i-1}=C_{pv}$, $R_i=R_{pv}$ et $L_i=0$.

8. Procédé selon l'une quelconque étant déterminé par des revendications 5-7, le modèle du compartiment central comprenant la structure suivante :

dans laquelle :
$p$ est la pression, $q$ est le débit, R est la résistance, *L est* l'inertance, C est la compliance, $X$ est la notion d'élastance variant dans le temps (E) ou de contrainte et de déformation des fibres myocardiques (MF), *R.A., R.V.* est l'atrium droit et le ventricule droit, *L.A., L.V.* est l'atrium gauche et le ventricule gauche, t.v. est la valve tricuspide (atrioventriculaire), *p.v.* est la valve pulmonaire (ventriculaire), *m.v.* est la valve mitrale (atrioventriculaire), *a.v.* est la valve aortique (ventriculaire), *pa* sont des artères (circulation pulmonaire), pv sont des veines (circulation pulmonaire), *sa* est l'aorte (circulation générale), et *sv* sont des veines (circulation générale).

9. Procédé selon l'une quelconque des revendications 5-6, le modèle du compartiment central comprenant un circuit en boucle fermée unique de circulation générale, dans lequel le bloc fonctionnel inertio-élastique est déterminé par $C_{i-1}=C_{sa}$, $R_i=R_{sa}$, $L_i=L_{sa}$, et le bloc fonctionnel résisto-capacitatif est déterminé par $C_{i-1}=C_{sv}$, $R_i=R_{sv}$ et $L_i=0$.

10. Procédé selon l'une quelconque des revendications 5-6 ou 9, le modèle du compartiment central comprenant la structure suivante :

dans laquelle :

$p$ est la pression, $q$ est le débit, R est la résistance, $L$ est l'inertance, C est la compliance, $X$ est la notion d'élastance variant dans le temps (E) ou de contrainte et de déformation des fibres myocardiques (MF), *L.A., L. V.* est l'atrium gauche et le ventricule gauche, m.v. est la valve mitrale (atrioventriculaire), *a.v.* est la valve aortique (ventriculaire), pv sont des veines (circulation pulmonaire), *sa* est l'aorte (circulation générale), et *sv* sont des veines (circulation générale).

11. Procédé selon l'une quelconque des revendications 5-10, le modèle du compartiment central comprenant des conditions suivantes :

$$q_i = \frac{(p_{i-1}-p_i)}{R_i} \text{ et } -\frac{dV}{dt} = q_i - q_{i-1}.$$

12. Procédé selon l'une quelconque des revendications 5-10, le modèle du compartiment central comprenant une relation de pressure-volume de chambre formulée en utilisant la notion d'élastance variable (E).

13. Procédé selon l'une quelconque des revendications 5-10, le modèle du compartiment central comprenant une relation de pressure-volume de chambre formulée en utilisant la notion de contrainte et de déformation des fibres myocardiques (MF).

14. Procédé selon l'une quelconque des revendications 1-13, l'identification paramétrique (4) du système couplé comprenant :

- Calculer des valeurs initiales des paramètres empiriques du système couplé en utilisant des données de santé et des données démographiques du ledit patient ;
- Résoudre des équations du système couplé en utilisant des valeurs initiales des paramètres empiriques pour calculer une valeur approximative de la pression artérielle centrale et/ou le débit proximale dans une fenêtre temporelle choisie ;
- Affiner de manière itérative (4.c) des valeurs des paramètres empiriques du système couplé par les valeurs constantes ou altérées de la pression artérielle centrale et/ou le débit proximale jusqu'à la convergence est atteinte.

15. Procédé selon la revendication 14, ledit calcul des valeurs initiales des paramètres empiriques du système couplé est réalisé seulement pour le compartiment central du modèle de paramètre groupé du système circulatoire sanguin.

16. Procédé selon l'une quelconque des revendications 14-15, lesdites paramètres empiriques comprenant la compliance, la résistance et/ou l'inertance et/ou lesdites paramètres empiriques comprenant des paramètres de la notion d'élastance variant dans le temps ou paramètres de la notion de contrainte et de déformation des fibres myocardiques.

17. Procédé selon l'une quelconque des revendications 14-16, ledit affinement itératif comprenant au moins un algorithme de minimisation, chacun ledit algorithme de minimisation est choisi dans le groupe constitué d'un algorithme de minimisation locale et d'un algorithme de minimisation globale.

18. Procédé selon la revendication 17, l'algorithme de minimisation locale est choisi dans le groupe constitué de la méthode de Nelder-Mead, l'optimisation des moindres carrés successive et la méthode de Broyden-Fletcher-Goldfarb-Shanno

19. Procédé selon l'une quelconque des revendications 16-18, l'algorithme de minimisation globale est choisi dans le groupe constitué de la méthode Adaptive Memory Programming for Global Optimization et de la méthode Simplicial Homology Global Optimization.

20. Procédé selon l'une quelconque des revendications 16-19, ledit affinement itératif comprenant une étape d'affinement qui implémente l'algorithme de minimisation globale suivi par une étape d'affinement qui implémente l'algorithme de minimisation locale.

21. Support de stockage lisible par ordinateur comprenant des instructions qui, lorsqu'elles sont exécutées par un ordinateur, amènent l'ordinateur à exécuter les étapes du procédé tel que défini dans l'une quelconque des revendications 1-20.

22. Système pour reconstruire d'une morphologie de forme d'onde de la pression artérielle centrale à partir d'une mesure distale continue non invasive de la pression, le système comprenant :

    - des moyens d'enregistrement pour l'enregistrement continu non invasif de forme d'onde de pression du patient humain ;
    - des moyens de mesurage pour mesurer de manière non invasive la pression systolique du patient, la pression diastolique du patient et la fréquence cardiaque du patient ;
    - Un ordinateur adapté à effectuer les étapes du procédé défini dans l'une quelconque des revendications 1-20.

FIG. 1

2WM: two-element Windkessel model
of Otto Frank

$q_{in}$
$p_{in}$

Rp

$q_{out}$
$p_{out}$

C

Frank O. Die Grundform des Arteriellen Pulses .
Zeitschrift für Biologie. 1899;37: 483-526

3WM: three-element Windkessel model
of Nicolaas Westerhof

$q_{in}$
$p_{in}$

Rc

Rp

$q_{out}$
$p_{out}$

C

Westerhof N et al. Analog studies of the human systemic arterial tree.
J Biomech. 1969; 2(2):121-143

4WM: four-element Windkessel model
of Nikos Stergiopulos

L

$q_{in}$
$p_{in}$

Rc

Rp

$q_{out}$
$p_{out}$

C

Stergiopulos N et al. Total arterial inertance as the fourth element of the windkessel model.
Am J Physiol. 1999; 276(1):H81-88

$p_{in}$, $q_{in}$ – pressure and flow rate at the inlet od compartment
$p_{out}$, $q_{out}$ – pressure and flow rate at the outlet od compartment
$R_c$, $R_p$, C, L – proximal and distal resistance, compliance and inertance

# FIG.2

**CRL:** building-block of lumped-parameter model

**1-CRL:** 1-compartment CRL lumped-parameter model

**2-CRL:** 2-compartment CRL lumped-parameter model

**n-CRL:** n-compartment CRL lumped-parameter model

$p, q$ -pressure and flow rate at inlet and outlet of compartments
$R, L, C$ -resistance, inertance, and compliance

# FIG.3

(a) vascular compartment functional block

(b) heart chamber functional block

$p, q$ -pressure and flow rate at inlet
and outlet of compartments
R, L, C -resistance, inertance,
and compliance
X - time-varying elastance (E) or
myocardial fiber stress and strain (MF)
of heart chamber

(c) double closed-loop central compartment circuit

(d) single closed-loop central compartment circuit

R.A., R.V. -right atrium and ventricle
L.A., L.V. -left atrium and ventricle
t.v. -tricuspid (atrio-ventricular) valve
p.v. -pulmonary (ventricular) valve
m.v. -mitral (atrio-ventricular) valve
a.v. -aortic (ventricular) valve
$pa$ -arteries (pulmonary circulation)
$pv$ -veins (pulmonary circulation)
$sa$ -aorta (systemic circulation)
$sv$ -veins (systemic circulation)

# FIG.4

FIG.5

FIG.6

## Convergence of Global Method of Central Pressure Reconstruction

## Convergence of Local Method of Central Pressure Reconstruction

FIG.7

FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20172018 B, Ostchega Y
- US 4869261 A
- US 10251566 B2, R Mukkamala
- US 2016196384 A
- US 2009287097 A
- US 2011137183 A
- WO 2020048642 A

**Non-patent literature cited in the description**

- *World Health Organization (WHO) guidelines*, 2021
- Guideline for the pharmacological treatment of hypertension in adults. *World Health Organization*, 2021
- **WHELTON PK**. ACC/ AHA/ AAPA/ ABC/ ACPM/ AGS/ APhA/ ASH/ ASPC/ NMA/ PCNA guideline for the prevention, detection, evaluation, and management of high blood pressure in adults: a report of the American College of Cardiology / American Heart Association Task Force on Clinical Practice Guidelines. *Hypertension*, 2017, vol. 71, e13-e115
- **WILLIAMS B**. ESC/ESH Guidelines for the management of arterial hypertension. *European heart journal*, 2018, vol. 39 (33), 3021-3104
- **ARONOW WS**. Hypertension and left ventricular hypertrophy. *Annals of Translational Medicine*, 2017, vol. 5 (15), 310
- **FOËX P**. Hypertension: pathophysiology and treatment. *Continuing Education in Anaesthesia Critical Care & Pain*, 2004, vol. 4 (3), 71-75
- **KOKUBO Y**. Higher blood pressure as a risk factor for diseases other than stroke and ischemic heart disease. *Hypertension*, 2015, vol. 66 (2), 254-259
- **BENJAMIN EJ**. Independent risk factors for atrial fibrillation in a population-based cohort. The Framingham Heart Study. *JAMA*, 1994, vol. 271 (11), 840-844
- Prevalence of hypertension in 1,795 subjects with chronic renal disease: the modification of diet in renal disease study baseline cohort. Modification of Diet in Renal Disease Study Group. **BUCKALEW VM**. American journal of kidney diseases: the official journal. National Kidney Foundation, 1996, vol. 28, 811-821
- **MORETTI R**. Risk factors for vascular dementia: hypotension as a key point. *Vascular health and risk management*, 2008, vol. 4 (2), 395-402
- **GORELICK PB**. Vascular contributions to cognitive impairment and dementia: A statement for healthcare professionals from the American Heart Association/American Stroke Association. *Stroke*, 2011, vol. 42 (9), 2672-2713
- **NOVAK V**. The relationship between blood pressure and cognitive function. *Nature reviews. Cardiology*, 2010, vol. 7 (12), 686-698
- **STOCKS T**. Blood pressure and risk of cancer incidence and mortality in the Metabolic Syndrome and Cancer Project. *Hypertension*, 2012, vol. 59 (4), 802-810
- *Hypertension*, 25 August 2021, https://www.who.int/news-room/fact-sheets/detail/hypertension
- **MUNTNER P**. Measurement of Blood Pressure in Humans: A Scientific Statement from the American Heart Association. *Hypertension*, 2019, vol. 73 (5), e35-e66
- **MCVEIGH GE**. Age-related abnormalities in arterial compliance identified by pressure pulse contour analysis: aging and arterial compliance. *Hypertension*, 1999, vol. 33 (6), 1392-1398
- **MILLASSEAU SC**. Determination of age-related increases in large artery stiffness by digital pulse contour analysis. *Clinical Science*, 2002, vol. 103 (4), 371-377
- **MACKENZIE IS**. Assessment of arterial stiffness in clinical practice. *QJM: Monthly Journal of the Association of Physicians*, 2002, vol. 95 (2), 67-74
- **SAUGEL B**. Cardiac output estimation using pulse wave analysis-physiology, algorithms, and technologies: a narrative review. *British Journal of Anaesthesia*, 2021, vol. 126 (1), 67-76
- **FAKLER U**. Cardiac index monitoring by pulse contour analysis and thermodilution after pediatric cardiac surgery. *The Journal of Thoracic and Cardiovascular Surgery*, 2007, vol. 133 (1), 224-228

- **ESPER SA**. Arterial waveform analysis. Best Practice & Research. *Clinical Anaesthesiology*, 2014, vol. 28 (4), 363-380
- **KANG HG**. Identification of Cerebral Artery Stenosis Using Bilateral Photoplethysmography. *Journal of Healthcare Engineering*, 2018, vol. 2018, 3253519
- **PICKERING TG**. Recommendations for blood pressure measurement in humans and experimental animals, part 1: blood pressure measurement in humans: a statement for professionals from the Subcommittee of Professional and Public Education of the American Heart Association Council on High Blood Pressure Research. *Hypertension*, 2005, vol. 45, 142-161
- **MIURA K**. Relationship of blood pressure to 25-year mortality due to coronary heart disease, cardiovascular diseases, and all causes in young adult men: the Chicago Heart Association Detection Project in Industry. *Archives of Internal Medicine*, 2001, vol. 161 (12), 1501-1508
- **MAREY EJ**. *Physiologie expérimentale: Travaux du Laboratoire de M. Marey*
- **MAUCK G**. The meaning of the point of maximum oscillations in cuff pressure in the indirect measurement of blood pressure--Part II. *Journal of Biomechanical Engineering*, 1980, vol. 102 (1), 28-33
- **AMOORE JN**. Can simulators evaluate systematic differences between oscillometric non-invasive blood-pressure monitors?. *Blood pressure monitoring*, 2000, vol. 5 (2), 81-89
- **WILLIAMS B**. ESC/ESH Guidelines for the management of arterial hypertension. *European Heart Journal*, 2018, vol. 39 (33), 3021-3104
- **LANE D**. Inter-arm differences in blood pressure: when are they clinically significant?. *Journal of Hypertension*, 2002, vol. 20 (6), 1089-1095
- **NETEA RT**. Influence of body and arm position on blood pressure readings: an overview. *Journal of Hypertension*, 2003, vol. 21 (2), 237-241
- **TERÉNT A**. Epidemiological perspective of body position and arm level in blood pressure measurement. *Blood pressure*, 1994, vol. 3 (3), 156-163
- **PETERS GL**. The effect of crossing legs on blood pressure: a randomized single-blind cross-over study. *Blood pressure monitoring*, 1999, vol. 4 (2), 97-101
- **LAKHAL K**. Noninvasive BP Monitoring in the Critically Ill: Time to Abandon the Arterial Catheter?. *Chest*, 2018, vol. 153 (4), 1023-1039
- **KARAMANOGLU**. M An analysis of the relationship between central aortic and peripheral upper limb pressure waves in man. *European Heart Journal*, February 1993, vol. 14 (2), 160-7
- **HOPE SA**. Generalizability' of a radial-aortic transfer function for the derivation of central aortic waveform parameters. *Journal of Hypertension*, 2007, vol. 25 (9), 1812-1820
- **CHEN CH**. Estimation of central aortic pressure waveform by mathematical transformation of radial tonometry pressure. Validation of generalized transfer function. *Circulation*, April 1997, vol. 1,95 (7), 1827-36
- **MIYASHITA H**. Clinical Assessment of Central Blood Pressure. *Current hypertension reviews*, 2012, vol. 8 (2), 80-90
- **GAO M**. A simple adaptive transfer function for deriving the central blood pressure waveform from a radial blood pressure waveform. *Scientific Reports*, 2016, vol. 6 (1), 1-9
- **FRANK P**. Noninvasive continuous arterial pressure monitoring during anesthesia induction in patients undergoing cardiac surgery. *Annals of Cardiac Anaesthesia*, 2021, vol. 24, 281-7
- **KIM SH**. Accuracy and precision of continuous noninvasive arterial pressure monitoring compared with invasive arterial pressure: a systematic review and meta-analysis. *Anesthesiology*, 2014, vol. 120 (5), 1080-1097
- **SAUGEL B**. Continuous noninvasive pulse wave analysis using finger cuff technologies for arterial blood pressure and cardiac output monitoring in perioperative and intensive care medicine: a systematic review and meta-analysis. *British Journal of Anaesthesia*, 2020, vol. 125 (1), 25-37
- **ILIES C**. Investigation of the agreement of a continuous non-invasive arterial pressure device in comparison with invasive radial artery measurement. *British Journal of Anaesthesia*, 2012, vol. 108 (2), 202-210
- **HAHN R**. Clinical validation of a continuous non-invasive haemodynamic monitor (CNAP™ 500) during general anaesthesia. *British Journal of Anaesthesia*, 2012, vol. 108 (4), 581-585
- **ZHOU S et al.** A review on low-dimensional physics-based models of systemic arteries: application to estimation of central aortic pressure. *Biomedical Engineering Online*, 2019, vol. 18 (1), 41, 1-25
- **MORRIS PD et al.** Computational fluid dynamics modelling in cardiovascular medicine. *Heart*, 2016, vol. 102, 18-28
- **RAINES JK** ; **JAFFRIN MY** ; **SHAPIRO AH**. A computer simulation of arterial dynamics in the human leg. *Journal of Biomechanics*, 1974, vol. 7 (1), 77-91
- **FORMAGGIA L** ; **LAMPONI D** ; **TUVERI M** ; **VENEZIANI A**. Numerical modeling of 1D arterial networks coupled with a lumped parameters description of the heart. *Computer Methods in Biomechanics and Biomedical Engineering.*, 2006, vol. 9 (5), 273-288

- **SWAMY G** ; **MUKKAMALA R** ; **OLIVIER N**. Estimation of the aortic pressure waveform from a peripheral artery pressure waveform via an adaptive transfer function. *Annual International Conference of the IEEE Engineering in Medicine and Biology Society*, 2008, 1385-1388
- **VLACHOPOULOS CH** ; **O'ROURKE M** ; **NICHOLS WW**. McDonald's Blood Flow in Arteries: Theoretical, Experimental and Clinical Principles. CRC Press, 2011
- **FRANK O.** Die Grundform des Arteriellen Pulses. *Zeitschrift für Biologie*, 1899, vol. 37, 483-526
- **WESTERHOF N et al.** Analog studies of the human systemic arterial tree. *Journal of Biomechanics.*, 1969, vol. 2 (2), 121-143
- **STERGIOPULOS N** ; **WESTERHOF BE** ; **WESTERHOF N**. Total arterial inertance as the fourth element of the Windkessel model. *Am J Physiol.*, 1999, vol. 276 (1), H81-88
- **BARRETT K et al.** Ganong's Review of Medical Physiology. McGraw-Hill Education, 2019
- **PAPPANO AJ** ; **WIER WG**. Cardiovascular Physiology. Elsevier, 2019
- **KLABUNDE RE**. Cardiovascular Physiology Concepts. Lippincott Williams & Wilkin, 2018
- **KIM HJ et al.** On coupling a lumped parameter heart model and a three-dimensional finite element aorta model. *Annals of Biomedical Engineering.*, 2009, vol. 37 (11), 2153-69
- **ITU L** ; **SHARMA P** ; **SUCIU C.** Patient-specific hemodynamic computations: application to personalized diagnosis of cardiovascular pathologies. Springer, 2017
- **HONGTAO L. et al.** A numerical model applied to the simulation of cardiovascular hemodynamics and operating condition of continuous-flow left ventricular assist device. *J. Mathematical Biosciences and Engineering*, 2020, vol. 17 (6), 7519-7543
- **CARO CG et al.** The Mechanics of the Circulation. Cambridge University Press, 2012
- **SUGA H.** Time course of left ventricular pressure-volume relationship under various enddiastolic volume. *Jpn Heart J.*, 1969, vol. 10 (6), 509-15
- **WALLEY KR**. Left ventricular function: time-varying elastance and left ventricular aortic coupling. *Critical Care*, 2016, vol. 20 (270), 1-11
- **BOZKURT S**. Mathematical modeling of cardiac function to evaluate clinical cases in adults and children.. *PloS One.*, 2019, vol. 14 (10), e0224663
- **LI W**. Biomechanics of infarcted left ventricle: a review of modelling. *Biomedical Engineering Letters*, 2020, vol. 10 (3), 387-417
- **MIROTA K**. Constitutive Models of Vascular Tissue. *Solid State Phenomena*, 2008, vol. 144, 100-105
- **AVAZMOHAMMADI R et al.** A Contemporary Look at Biomechanical Models of Myocardium. *Annual Review of Biomedical Engineering.*, 04 June 2019, vol. 21, 417-442
- **VOIGT JU** ; **CVIJIC M**. 2- and 3-Dimensional Myocardial Strain in Cardiac Health and Disease. *JACC Cardiovasc Imaging.*, 2019, vol. 12 (9), 1849-1863
- **SMULYAN H et al.** Influence of body height on pulsatile arterial hemodynamic data.. *Journal of the American College of Cardiology.*, 1998, vol. 31 (5), 1103-9
- **CHRISTOFARO DGD et al.** Relationship between Resting Heart Rate, Blood Pressure and Pulse Pressure in Adolescents. *Arquivos Brasileiros de Cardiologia.*, 2017, vol. 108 (5), 405-410
- **EVANS JM et al.** Body Size Predicts Cardiac and Vascular Resistance Effects on Men's and Women's Blood Pressure. *Front Physiol.*, 2017, vol. 9 (8), 561
- **GALLO C et al.** Testing a Patient-Specific In-Silico Model to Noninvasively Estimate Central Blood Pressure.. *Cardiovascular Engineering and Technology.*, 2021, vol. 12 (2), 144-157
- **HARRIS WS** ; **SCHOENFELD CD** ; **WEISSLER AM**. Effects of adrenergic receptor activation and blockade on the systolic preejection period, heart rate, and arterial pressure in man.. *Journal of Clinical Investigation.*, 1967, vol. 46 (11), 1704-14
- **MORGAN TO et al.** A comparison of beta adrenergic blocking drugs in the treatment of hypertension.. *Postgraduate Medical Journal*, 1974, vol. 50 (583), 253-259
- **NYBERG G**. Effect of beta-adrenoreceptor blockers on heart rate and blood pressure in dynamic and isometric exercise.. *Drugs*, 1976, vol. 11 (1), 185-95
- **FITZPATRICK MA** ; **JULIUS S**. Hemodynamic effects of angiotensin-converting enzyme inhibitors in essential hypertension: a review.. *Journal of Cardiovascular Pharmacology*, 1985, vol. 7 (1), 35-9
- **TING CT**. Arterial hemodynamics in human hypertension. Effects of angiotensin converting enzyme inhibition.. *Hypertension.*, 1993, vol. 22 (6), 839-46
- **JOBS A et al.** Angiotensin-converting-enzyme inhibitors in hemodynamic congestion: a meta-analysis of early studies.. *Clinical Research in Cardiology.*, 2019, vol. 108 (11), 1240-1248
- **BLOCK PJ** ; **WINKLE RA**. Hemodynamic effects of antiarrhythmic drugs.. *American Journal of Cardiology.*, 1983, vol. 52 (6), 14C-23C
- **WEINER B**. Hemodynamic effects of antidysrhythmic drugs.. *Journal of Cardiovascular Nursing.*, 1991, vol. 5 (4), 39-48

- **RODRIGUEZ-MOLINERO A**. Normal respiratory rate and peripheral blood oxygen saturation in the elderly population. *Journal of the American Geriatrics Society*, 2013, vol. 61 (12), 2238-2240
- **PARK C** ; **LEE B**. Real-time estimation of respiratory rate from a photoplethysmogram using an adaptive lattice notch filter. *Biomedical Engineering Online*, 2014, vol. 17 (13), 170
- **SCHOLKMANN F** ; **WOLF U**. The Pulse-Respiration Quotient: A Powerful but Untapped Parameter for Modern Studies About Human Physiology and Pathophysiology. *Front Physiol.*, 2019, vol. 9 (10), 371
- **WALTER E**. Identification of Parametric Models: from Experimental Data. Springer, 1997
- **BOCK HG**. Model Based Parameter Estimation. Springer, 2013
- **KHOO M**. Physiological Control Systems: Analysis, Simulation, and Estimation. John Wiley & Sons, 2018
- **BITTANTI S**. Model Identification and Data Analysis. John Wiley & Sons, 2019
- **VILLAVERDE AF et al.** Benchmarking optimization methods for parameter estimation in large kinetic models. *Bioinformatics*, 2019, vol. 35 (5), 830-838
- **KREUTZ C**. Guidelines for benchmarking of optimization-based approaches for fitting mathematical models. *Genome Biol.*, 2019, vol. 20 (1), 281
- **SCHMIESTER L**. Efficient parameterization of large-scale dynamic models based on relative measurements. *Bioinformatics*, 2020, vol. 36 (2), 594-602
- **NELDER J** ; **MEAD R**. A simplex method for function minimization. *Computer Journal*, 1965, vol. 7 (4), 308-313
- **GAO F** ; **HAN L**. Implementing the Nelder-Mead simplex algorithm with adaptive parameters. *Computational Optimization and Applications*, 2010, vol. 51 (1), 259-277
- **KRAFT D**. A software package for sequential quadratic programming.. DFVLR, 1988
- **ZHU C** ; **BYRD RH** ; **NOCEDAL J**. Algorithm 778. L-BFGSB: Fortran routines for large scale bound constrained optimization.. *ACM Transactions on Mathematical Software*, 1997, vol. 23 (4), 550-560
- **LASDON L et al.** Adaptive memory programming for constrained global optimization.. *Computers & Operations Research*, 2010, vol. 37 (8), 1500-1509
- **ENDRES S**. A simplicial homology algorithm for Lipschitz optimization. Department of Chemical Engineering, University of Pretoria, 2017
- Topological structure of finite element models of continuum mechanics. **MIROTA K**. Bulletin of the Military University of Technology. 2008, vol. LVII, 91-102